# EUROPEAN PATENT SPECIFICATION

(11) **EP 0 839 044 B1**
(45) Date of publication and mention of the grant of the patent: **18.05.2005**
(21) Application number: 96911493.3
(22) Date of filing: 29.03.1996
(51) Int. Cl.: A61K 35/14, C12N 5/22, C12N 15/07, C12N 5/06

(54) **DENDRITIC-LIKE CELL/TUMOR CELL HYBRIDS FOR INDUCING AN ANTI-TUMOR RESPONSE**
DENDRITENZELLEN/TUMORZELLEN Hybirde ZUR INDUKTION EINER ANTI-TUMOR ANTWORT
CELLULES DENDRITIFORMES/CELLULES TUMORALES HYBRIDES DESERVANT A PROVOQUER UNE REPONSE ANTITUMORALE

(30) Priority: 31.03.1995 US 414480
(43) Date of publication of application: 06.05.1998
(73) Proprietor: UNIVERSITE LIBRE DE BRUXELLES, 1050 Bruxelles (BE); VRIJE UNIVERSITEIT BRUSSEL, 1050 Brussel (BE)
(72) Inventor: MOSER, Muriel, B-1970 Wezembeek-Oppem (BE); LEO, Oberdan, B-1970 Wezembeek-Oppem (BE); LESPAGNARD, Laurence, B-1050 Bruxelles (BE); URBAIN, Jacques, B-1380 Lasne (BE); BRUYNS, Catherine, B-1640 Rhode-St-Genese (BE); GERARD, Catherine, B-1150 Brussels (BE); GOLDMAN, Michel, B-1180 Brussels (BE); VELU, Thierry, B-1180 Brussels (BE); WILLEMS, Fabienne, B-1630 Linkebeek (BE); TASIAUX, Nicole, B-1169 Bruxelles (BE); PERRET, Jason, B-1070 Brussels (BE); VERHEYDEN, Anne-Marie, B-1300 Wavre (BE); METTENS, Pascal, B-1030 Bruxelles (BE); Thielemans, Kris, B-1860 Meise (BE)
(74) Representative: De Clercq, Ann
(86) International application number: PCT/US1996/004370
(87) International publication number: WO 1996/030030

(56) References cited:
- PETERS J H: "DENDRITIC CELL HYBRIDOMA ACTION ON T LYMPHOCYTE PROLIFERATION" IMMUNOBIOLOGY, vol. 159, no. 1-2, 1981, page 159 XP001000956 ISSN: 0171-2985
- NAGASAKI MAKOTO ET AL: "A new monoclonal antibody (IE8) reactive with dendritically shaped cells in the human tonsil." PATHOLOGY INTERNATIONAL, vol. 45, no. 4, 1995, pages 266-274, XP000100961 ISSN: 1320-5463
- EZQUERRA A ET AL: "MOUSE AUTOREACTIVE GAMMA-DELTA T CELLS II. MOLECULAR CHARACTERIZATION OF THE T CELL RECEPTOR" EUROPEAN JOURNAL OF IMMUNOLOGY, vol. 22, no. 2, 1992, pages 491-498, XP001000937 ISSN: 0014-2980
- STUHLER G ET AL: "RECRUITMENT OF HELPER T CELLS FOR INDUCTION OF TUMOUR REJECTION BY CYTOLYTIC T LYMPHOCYTES" CANCER IMMUNOLOGY AND IMMUNOTHERAPY,BERLIN,DE, vol. 39, no. 5, 1994, pages 342-345, XP000993203 ISSN: 0340-7004
- GUO ET AL: 'Effective Tumor Vaccine Generated by Fusion of Hepatoma Cells with Activated B Cells' SCIENCE vol. 263, 28 January 1994, pages 518 - 520, XP002167016
- JOURNAL OF EXPERIMENTAL MEDICINE, Volume 178, issued December 1993, PAGLIA et al., "Immortalized Dendritic Cell Line Fully Competent in Antigen Presentation Intiates Primary T Cell Responses In Vivo", pages 1893-1901, XP000572922
- JOURNAL OF CLINICAL INVESTIGATION, Volume 85, issued March 1990, MARKOWITZ et al., "Granulocyte-Macrophage Colony-Stimulating Factor Promotes Differentiation and Survival of Human Peripheral Blood Dendritic Cells In Vitro", pages 955-961, XP000386310
- JOURNAL OF EXPERIMENTAL MEDICINE, Volume 172, issued August 1990, INABA et al., "Dendritic Cells Pulsed with Antigens in Vitro Can Prime Antigen-Specific, MHC-Restricted T Cells in Situ", pages 631-640, XP000569394
- PROC. NATL. ACAD. SCI. U.S.A., Volume 85, issued August 1988, HAUSER et al., "Activation and Expansion of Hapten- and Protein-Specific T Helper Cells from Nonsensitized Mice", pages 5625-5628, XP002946708
- JOURNAL OF EXPERIMENTAL MEDICINE, Volume 183, issued January 1996, ZITVOGEL et al., "Therapy of Murine Tumors with Peptide-Pulsed Dendritic Cells: Dependence on T Cell, B7 Costimulation and T Helper Cells 1-Associated Cytokines", pages 87-97, XP002910558
- THE JOURNAL OF IMMUNOLOGY, Volume 146, issued 15 May 1991, GRABBE et al., "Tumor Antigen Presentation by Murine Epidermal Cells", pages 3656-3661, XP002946757
- ANNUAL REVIEW OF IMMUNOLOGY, Volume 9, issued 1991, STEINMAN, "The Dendritic Cell System and its Role in Immunogenicity", pages 271-296, XP000605744
- JOURNAL OF EXPERIMENTAL MEDICINE, Volume 176, issued December 1992, INABA et al., "Generation of Large Numbers of Dendritic Cells from Mouse Bone Marrow Cultures Supplemented with Granulocyte/Macrophage Colony-Stimulating Factor", pages 1693-1702, XP000386312
- JOURNAL OF EXPERIMENTAL MEDICINE, Volume 175, issued May 1992, INABA et al., "Identification of Proliferating Dendritic Cell Precursors in Mouse Blood", pages 1157-1167, XP002946758

## Description

### Technical Field

This invention is in the field of immunotherapy for the treatment of cancer. Specifically, the invention relates to the use of a hybridoma consisting of a fused tumor cell and a dendritic cell, which hybridoma is capable of inducing an anti-tumor response in vivo when administered to a subject in need of anti-tumor treatment.

### Background of the Invention

### The Immune Response

The introduction of pathogens such as bacteria, parasites or viruses into a mammal elicits a response contributing to the specific elimination of the foreign organism Foreign material is referred to as antigen, and the specific response is called the immune response. The immune response starts with the recognition of the antigen by a lymphocyte, proceeds with the elaboration of specific cellular and humoral effectors and ends with the elimination of the antigen by the specific effectors. The specific effectors are essentially T lymphocytes and antibodies, mediating cellular and humoral immune responses, respectively. The present invention relates to the initiation of a cellular immune response. The initiation of a cellular immune response starts with the recognition of an antigen on the surface of an antigen-presenting cell (APC).

### Antigen Recognition by T-Lymphocytes

Cellular antigen recognition is operated by a subset of lymphocytes called T-lymphocytes. T-lymphocytes include two major functional subsets. They are T-helper lymphocytes (TH), that usually express the CD4 surface marker, and cytotoxic T-lymphocytes (CTL), that usually express the CD8 surface marker. Both T-cell subsets express an antigen receptor that can recognize a given peptide antigen. The peptide needs to be associated with a major histocompatibility molecule (MHC) expressed on the surface of the APC, a phenomenon known as MHC restriction. T-cells bearing the CD4 surface marker recognize peptides associated with MHC class II molecules, whereas T-cells bearing the CD8 surface marker recognize peptides associated with MHC class I molecules.

Since the T-cell antigen receptor can only recognize peptides associated with MHC molecules at the surface of an APC, cellular proteins need to be processed into such peptides and transported with MHC molecules to the cell surface. This is referred to as antigen processing. Exogenous proteins, phagocytosed by the APC, are broken down into peptides that are transported on MHC class II molecules to the cell surface, where they can be recognized by CD4⁺ T-cells. In contrast, endogenous proteins, synthesized by the APC, are also broken down into peptides, but the latter are transported on MHC class I molecules to the cell surface, where they can be recognized by CD8⁺ T-cells.

When a T-cell binds through its antigen receptor to its cognate peptide-MHC complex on an APC, the binding generates a first signal from the T-cell membrane towards its nucleus. However, this first signal is insufficient to activate the T-cell, at least as measured by the induction of IL-2 synthesis and secretion. Activation only occurs if a second signal or costimulatory signal is generated by the binding of other APC surface molecules to their appropriate receptors on the T-cell surface. The best known costimulatory molecules identified to date on APC are B7-1 (Razi-Wolf et al, 1993, Proc. Natl. Acad. Sci. USA 90: 11182-1186) and B7-2 (Hathcock et al, 1993, Science 262: 905-907); both bind to the CD28/CTLA4 counter-receptor on T-lymphocytes. The capacity to present peptide antigens together with costimulatory molecules in such a way as to activate T-cells is hereafter referred to as antigen presentation. Only APCs have the capacity to present antigens to CD4⁺ (predominantly TH) and CD8⁺ (predominantly CTL) T-cells, leading to the development of humoral and cellular immune responses.

### T-lymphocyte Activation by Antigen-Presenting Cells

APCs are heterogeneous in their cell lineage and functional performance. They include distinct cell types such as B lymphocytes, T lymphocytes, monocytes/ macrophages and dendritic cells from myeloid origin. All these cells are bone marrow-derived cells, that need to mature and to be activated in order to function efficiently as APCs.

The functional performances of APCs rely critically upon the nature and state of maturation of the cells included in purified or enriched APC preparations. The latter vary with the tissue of origin and method of purification. In an operational way, we call dendritic-like cells (DLCs) ) all non-B cells present in purified or enriched preparations of dendritic cells. These cells all share some morphological, physical or biochemical characteristics with dendritic cells, leading to their co-purification with dendritic cells. Therefore, the term DLCs refers hereafter not only to dendritic cells of myeloid origin, but also to monocytes, T-lymphocytes and other non-Bcells present in enriched or purified dendritic cell preparations. In mice, the spleen is very often used as a source of DLCs (reviewed by Steinman, 1991, Annu. Rev Immunol 9: 271-296). However, mouse DLCs have also been generated by in vitro culture from bone marrow progenitors in the presence of cytokines (Inaba et al, 1992, J. Exp. Med. 176: 1693-1702). In humans, blood or bone marrow are the usual sources of DLCs, that are used either immediately or more often after culture in the presence of cytokines. Several protocols of purification and in vitro culture have been published (reviewed in Young and Inaba, 1996, J. Exp. Med 183: 7-11), and patent applications have been filed for some of them (WO 93/20185, by Steinman R., Inaba K. Schuler G WO 93/20186, by Banchereau J and Caux C; WO 94/02156 by Engleman E.., Markowicz S and Metha A; WO 95/28479 by Brugger W and colleagues of Mertelsmann R.).

### T-Lymphocyte Activation by Tumor Cells

There is increasing evidence that tumor cells do not usually function as APCs (reviewed by Young and Inaba, 1996, J. Exp. Med 183: 7-11). Although some tumor cells are capable of delivering an antigen-specific signal to T cells, they may not provide the costimulatory signals which are necessary for the full activation of T-cells and thereby fail to induce an efficient anti-tumor immune response. In order to compensate for this inefficient induction of an anti-tumor immune response, different approaches have been tried in experimental animals (reviewed by Grabbe et al, 1995, Immunology Today 16: 117-121).

In one such approach, tumor cells were genetically engineered to express one or more molecules known to be involved in antigen presentation on APC. To date, efficient in vivo results from this approach were obtained with tumor cells co-expressing MHC class I, MHC class II and B7-1 molecules, suggesting that the successful immunotherapy was linked to the activation of both CD4⁺ and CD8⁺ T cells. For example, Basker et al, 1995, J. Exp. Med 181:619-629 engineered mouse fibrosarcoma cells, that naturally express MHC class I molecules, to express in addition MHC class II and B7-1 molecules; the injection of these modified tumor cells was sufficient to cure syngeneic mice carrying large established tumors. It should be noted that tumor cells expressing MHC class I but not MHC class II molecules and transduced with the B7-1 costimulator also induced an in vivo anti-tumor immune response, and that the latter depended upon the activation of CD8⁺, but not CD4⁺ T cells (Ramarathinam et al, 1994, J. Exp. Med. 179: 1205-1214). The disadvantage of this approach lies in the genetic engineering of the tumor cells, a technique that usually involves the use of viral vectors for efficient gene transfer. Viral vectors are not totally safe for the treatment of human patients. The main reason is that they can recombine both in vitro and in vivo, which may lead to the production of novel wild type viruses of unpredictable pathogenicity. This limitation stimulated the development of alternative methods of efficient gene transfer, such as the one recently described by Birnstiel and colleagues (WO 94/21808).

In another approach, APCs were loaded with a source of tumor antigens. Amongst the APC tested for such a purpose, DLCs appeared to be the most efficient. To date, it is clear that DLCs pulsed with tumor cell lysates (Knight et al, 1985, Proc. Natl. Acad. Sci. USA 82: 4495-4497), with a purified tumor-associated protein (Flamand et al, 1994, Eur. J. Immunol 24: 605 - 610; Paglia et al, 1996, J. Exp. Med. 183: 317-322) or with tumor-associated peptides (Ossevoort et al, 1995, J. Immunotherapy 18: 86-94; Mayordomo et al, 1995, Nature Medicine, 1: 1297-1302) can efficiently induce an anti-tumor immune response in vivo There are, however, disadvantages to this approach. Tumor cell lysates or fractions thereof are relatively easy to prepare, but the loading of DLCs with such crude preparations could, at least theoretically, induce adverse auto-immune reactions in the host. Similar secondary effects could be induced by DLCs loaded with all the peptides eluted from tumor cells, as described by Zitvogel et al, J. Exp. Med. 1996, 183: 87-97. The latter risk is reduced by pulsing DLCs with purified, tumor-specific antigens or peptides. However, there are very few known tumor-specific antigens, and in addition, their production and purification are both labor-intensive and expensive.

In a recent approach, a tumor cell and one sort of APC, namely a B-lymphocyte, were united into a single cell by somatic cell fusion (Guo et al, 1994, Science 263: 518-520). Guo et al. fused a rat hepatoma cell line with in vivo-activated B lymphocytes, and showed that some of the resulting B cell/tumor cell hybridomas induced tumor-resistance in syngeneic rats and also cured the animals of a small pre-established tumor. The selected hybridomas expressed MHC class II restriction elements and B7 costimulatory molecules, which strongly suggested that the immunotherapy worked through the activation of CD4⁺ TH cells. When compared to the two previous approaches, this third approach has the general advantages of somatic cell fusion, namely, it brings together not only the known tumor antigens and known costimulators of activated B-cells, but possibly some as yet unknown molecules carrying out these functions. When compared to the genetic engineering of tumor cells, this cellular engineering does not require the identification of the genes encoding costimulatory molecules, nor their transfer into tumor cells. Similarly, when compared to the pulsing of APC with purified tumor-specific antigens, somatic cell fusion does not require the identification of genes encoding tumor-specific antigens, nor the production and purification of the corresponding recombinant proteins. However, in its present description, this approach is inapplicable to human cancer patients, because it involves the use of in vivo-activated B-cells as fusion partners of the tumor cells. In vivo-activated B-cells were recovered from the spleen forteen days after immunization with soluble antigen in complete Freund's adjuvant, which cannot be used in humans. In addition, if immunizations are done without Freund's adjuvant, the outcome of an in vivo activation of B-cells remains unpredictable in individual animals, and it is expected to be unpredictable in individual human patients. Finally, the selection of the hybridomas is quite labor-intensive. It required the preparation, absorption and characterization of tumor-specific polyclonal antisera, that were used to select the cells expressing surface markers of the tumor parent; this first selection was then followed by a second selection of cells expressing surface markers of the in vivo-activated B-cell parent.

What is really needed is a method to harness the ability of DLCs to elicit an anti-tumor response, so that the immune system of a subject can mount a rejection of the tumor cells. In addition, this method should be transposable to human cancer patients.

### Summary of the Invention

The present invention relates to the use of DC/tumor cell hybridomas and a plurality of DC/tumor cell hybrids in the treatment of cancers. Said hybridomas and hybrids are capable of inducing an anti-tumor response when administered to the subject, in vivo.

In a first embodiment, the present invention relates to the use of a plurality of dendritic cell/tumor cell hybrids for the preparation of a medicament for producing an anti-tumor response in a mammalian subject.

In a second embodiment, the present invention relates to the use of a dendritic cell/tumor cell hybridoma for the preparation of a medicament for producing an anti-tumor response in a mammalian subject.

According to the present invention, said dendritic cell and/or the tumor cell may be human in origin.

In addition, said dendritic cell may be derived from bone marrow, may be of myeloid origin or lymphoid origin; or; may be an isolated dendritic cell or a dendritic cell progenitor.

According to the present invention the dendritic cell/tumor hybrids used according to the present invention may be produced using a method comprising:
(a) providing a sample of a tumor against which said response is needed,
(b) preparing a primary cell culture comprising tumor cells derived from said tumor sample,
(c) providing autologous, HLA-compatible or allogeneic dendritic cells, and,
(d) fusing said dendritic cells with said tumor cells to produce a plurality of hybrids.

Preferentially, in the above-mentioned method the dendritic cells of step (c) are produced by culturing precursors in the presence of cytokines. Furthermore; the dendritic cells of step (c) may be members of an immortal cell line.

The dendritic cell/tumor hybridoma used according to the present invention may be produced using a method comprising:
(a) providing a sample of a tumor against which said response is needed,
(b) preparing a primary culture of said tumor sample to provide tumor cells,
(c) deriving an immortal cell line from said tumor cells to produce immortal tumor cells,
(d) providing autologous or HLA-compatible allogeneic dendritic cells,
(e) fusing said dendritic cells with said immortal tumor cells to produce a plurality of hybridomas,
(f) selecting from said plurality of hybridomas a hybridoma which exhibits at least one characteristic selected from the group consisting of dendritic cell morphology, dendritic-like cell or dendritic cell surface markers, dendritic cell genetic markers and immune cell activation *in vitro*.

In the above-mentioned method, the dendritic cells of step (d) may be produced by culturing precursors in the presence of cytokines. In addition, the immortal tumor cells of step (c) may be drug-sensitive, said method further comprising, after step (e), killing unfused drug-sensitive immortal tumor cells by exposure to said drug.

The dendritic cell/tumor hybridoma used according to the present invention may also be produced using a method comprising:
(a) providing a sample of a tumor against which said response is needed,
(b) preparing a primary cell culture comprising tumor cells derived from said tumor sample,
(c) providing an immortal cell line comprising immortal autologous or HLA-compatible allogeneic dendritic cells,
(d) fusing said immortal dendritic cells with said tumor cells to produce a plurality of hybridomas, and
(e) selecting from said plurality of hybridomas, a hybridoma which exhibits at least one characteristic selected from the group consisting of tumor cell morphology, tumor cell surface markers, tumor cell chromosomal and genetic markers.

Said above-mentioned method may further comprise a step for selecting from said plurality of hybridomas, a hybridoma which exhibits at least one characteristic selected from the group consisting of dendritic cell morphology, dendritic cell surface markers, dendritic cell genetic markers and immune cell activation *in vitro*.

In addition, in the above-mentioned method the dendritic cells of step (c) may be drug sensitive, said method further comprising, after step (d), killing unfused drug-sensitive immortal dendritic cells by exposure to said drug.

Alternatively, the dendritic cell/tumor hybridoma used according to the present invention may also be produced using a method comprising:
(a) providing a sample of a tumor against which said response is needed,
(b) analyzing tumor-associated antigens of said tumor sample,
(c) providing an established cell line comprising immortal human tumor cells having at least one tumor-associated antigen in common with said tumor sample,
(d) providing autologous, HLA-compatible or allogeneic dendritic cells,
(e) fusing said dendritic cells with said immortal tumor cells to produce a plurality of hybridomas, and
(f) selecting from said plurality of hybridomas, a hybridoma which exhibits at least one characteristic selected from the group consisting of dendritic cell morphology, dendritic cell surface markers, dendritic cell genetic markers and immune cell activation *in vitro*.

The above-mentioned method may further comprise a step for selecting from said plurality of hybridomas, a hybridoma which expresses at least one tumor-associated antigen in common between the immortal tumor cells and the tumor against which an immune response is needed.

In addition, in the above-mentioned method the dendritic cells of step (d) may be produced by culturing in the presence of cytokines. Furthermore, the tumor cells of step (c) may be drug sensitive, said method comprising, after step (e), killing unfused drug-sensitive tumor cells by exposure to said drug.
Said drug may be hypoxanthine-aminopterin-thymidine (HAT).

According to the invention, the hybrid or hybridoma obtained by a method as described above may be further induced to express the dendritic cell characteristics. Said dendritic cell characteristics may be chosen from the group consisting of dendritic cell morphology, dendritic cell surface markers or dendritic cell activation markers and immune cell activation properties *in vitro*. The present invention further specifies that said induction may be performed using GM-CSF.

In addition, the present invention further specifies that the hybrids or hybridoma obtained by a method as described above may be further treated to prevent proliferation before using it for the induction of an anti-tumor response. Said treatment may occur by irradiation.

A DC/tumor cell hybridoma of the invention is produced by first providing a sample of the specific tumor against which an immune response is needed. In one embodiment of the invention, an immortal cell line is derived from the tumor sample, and then the tumor cells are fused with DCs. Preferably, autologous DCs from the subject are used, but matched HLA-compatible DCs may also be used as fusion partners. Once the DCs are fused with the tumor cells, selection is carried out. In this embodiment, hybridomas which exhibit DCs characteristics are selected, their immortality being necessarily contributed by fusion with the tumor cell. In a second embodiment of the invention, an established immortal human tumor cell line is provided which expresses at least one of the tumor-associated antigens of the patient's tumor cells. Cells from the tumor cell line are fused with autologous or HLA-compatible allogeneic DCs to form hybridomas which are then selected for retention of DC characteristics. In a third embodiment of the invention, an immortal DC line is established, and then DCs of this line are fused with the patient's tumor cells from primary culture. The resulting hybridomas are selected for retention of DC characteristics as well as expression of at least one tumor-associated antigen of the patient's tumor cells.

In other embodiments of the invention, tumor cells are fused with DCs, and the resulting plurality of hybrids is used directly for treatment, without selection.

The DC/tumor cell hybridoma, or plurality of hybrids, is administered to the subject to induce an immune response against residual tumor cells in the subject's circulation or organs. Alternatively, the hybridoma or plurality of hybrids is co-cultivated in vitro with immune cells from the subject in order to activate against the tumor cell; the activated immune cells are then returned to the subject.

### Brief Description of the Drawings

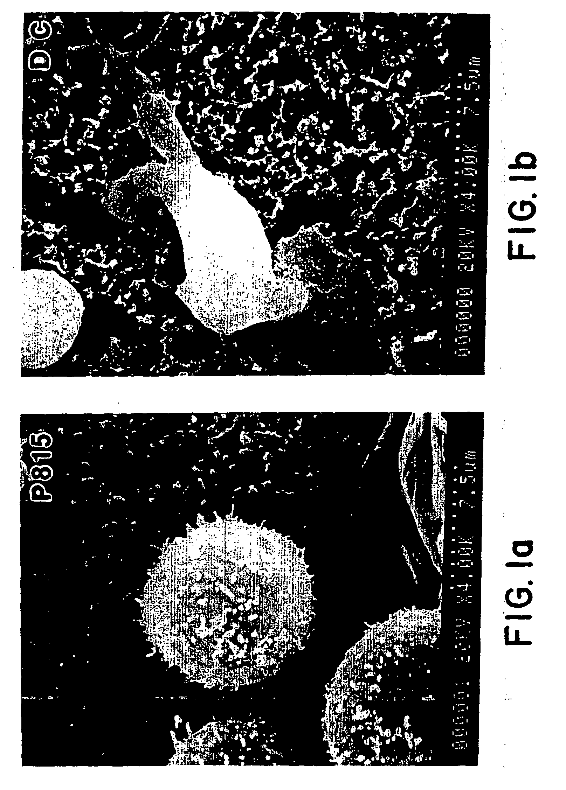

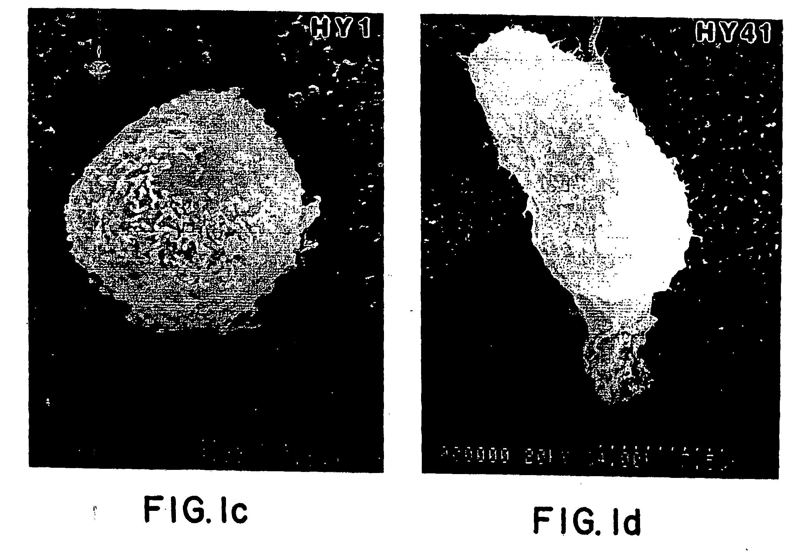

Figure 1. Scanning electron microscopy of parent cells and of two murine DLC/tumor cell hybridomas (x 4,000). The figure illustrates the "tumor-like" and "dendritic-like"characteristics of two DLC/tumor cell hybridomas. Hybridoma HY1 (Fig. 1c) resembles more the parent P815* tumor cell (Fig. 1a) than the parent dendritic cell (Fig. 1b), whereas hybridoma HY41 (Fig. 1d) resembles more the dendritic cell (Fig. 1b) than the P815* tumor cell (Fig. 1a). It is the "dendritic-like" hybridoma HY41 that was selected for in vivo experiments.

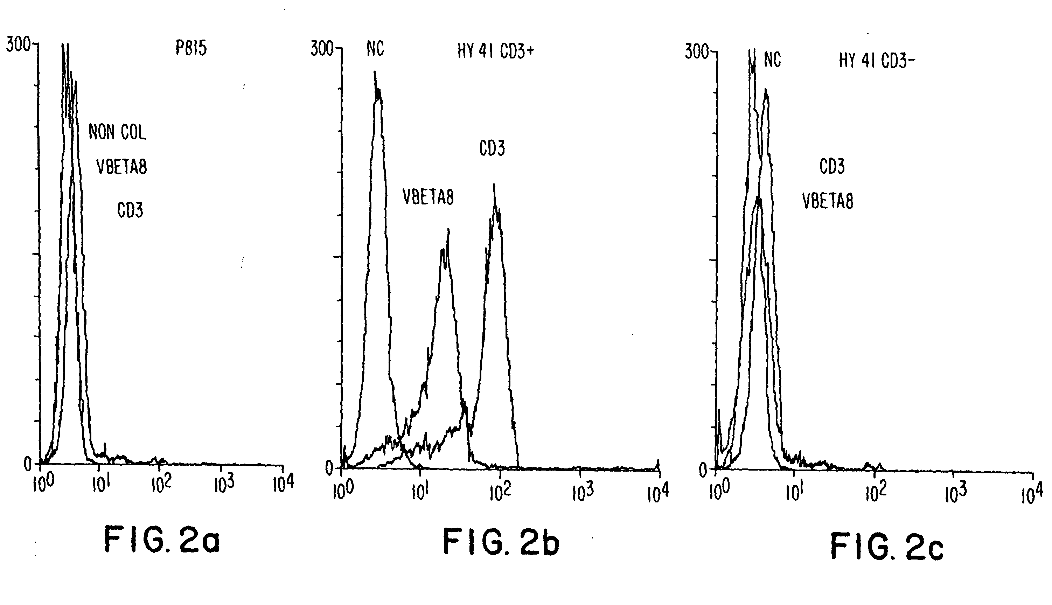

Figure 2a-e. FACS analysis of DLC/tumor cell hybridomas HY41 and HY62, showing the expression of CD3 and the TCR V-β8 domain by the CD3-positive subclones (HY41 CD3+ and HY62 CD3+); the CD3-negative subclones of these hybridomas (HY41 CD3- and HY62 CD3-) as well as the parent P815* tumor cells fail to express the TCR V-β8 domain.

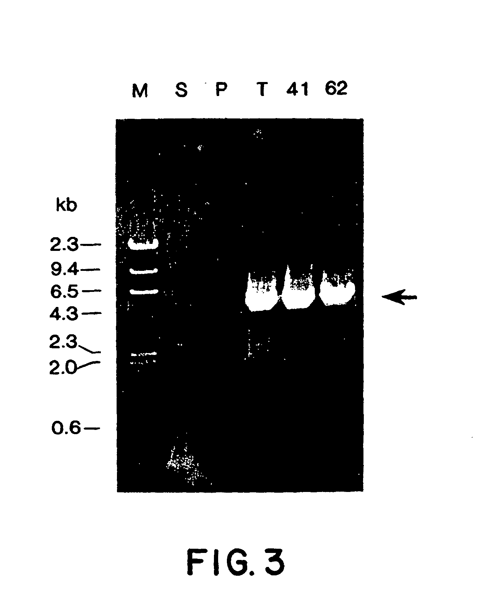

Figure 3. Ethidium bromide-stained gel electrophoresis of Polymerase Chain Reaction products obtained with mouse genomic DNA, using TCR V-β8 and Cβ primers. A rearranged TCRβ gene fragment was amplified from genomic DNA of a mouse T-cell hybridoma (T), as well as from the HY41 (41) and HY62 (62) DLC/tumor cell hybridomas; no rearranged TCRβ fragment was amplified from DNA of P815* tumor cells (P) and spleen cells (S), used as negative controls.

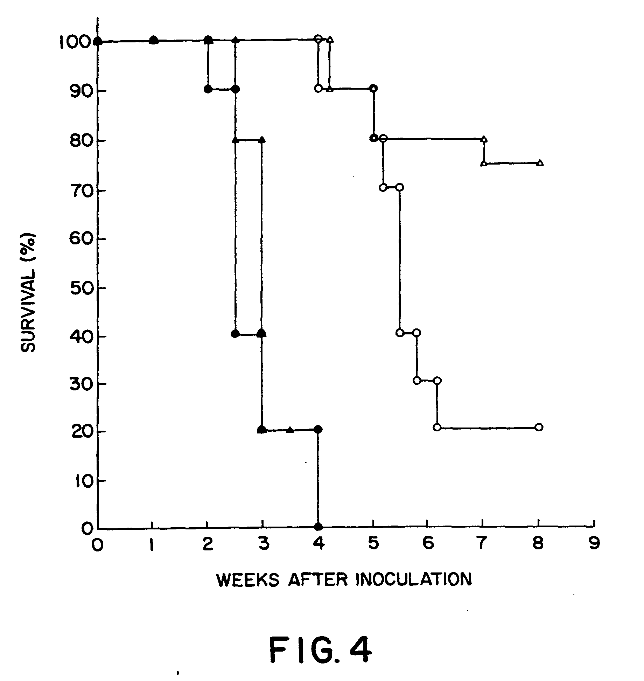

Figure 4. Survival curves of immunocompetent and immunocompromised (i.e. irradiated) DBA/2 mice after ip inoculation with 5 x 10⁵ syngeneic hybridoma cells HY41 or with the same number of parental P815* tumor cells.
   - Key:: ○ P815 in normal mice (n = 10)
   ● P815 in irradiated mice (n = 10)
   Δ HY 41 in normal mice (n = 12)
   ▲ HY 41 in irradiated mice (n = 10)
   This figure shows that the "dendritic-like" hybridoma HY41 was rejected by 75% (9/12) of the immunocompetent mice, while the parent tumor was rejected, in this particular experiment, by 20% (2/10) of the animals. This difference in survival was not due to a difference in tumorigenicity, since both cell lines killed all (10/10) immunocompromised animals within four weeks of inoculation.

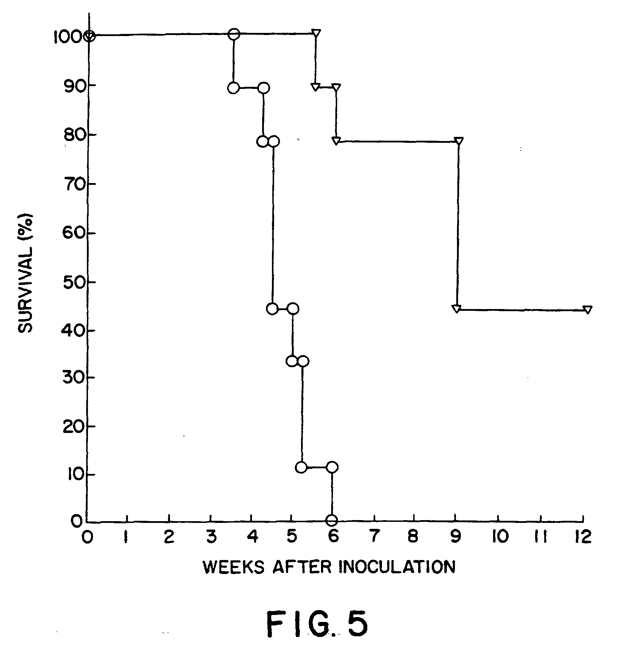

Figure 5. Survival curves of naive and HY41-treated DBA/2 mice after ip inoculation with 5 x 10⁵ syngeneic P815* tumor cells. Y-axis = survival (%). X-axis = weeks after inoculation.
   - Key:: ○ normal mice (n = 9)
   ∇ "HY41" - treated mice (n = 9)
   This figure shows that the nine HY41-survivors (see Fig. 4) became at least partially resistant to a lethal challenge with the parental P815* tumor cells, and that 4/9 of these animals showed complete tumor resistance for at least three months.

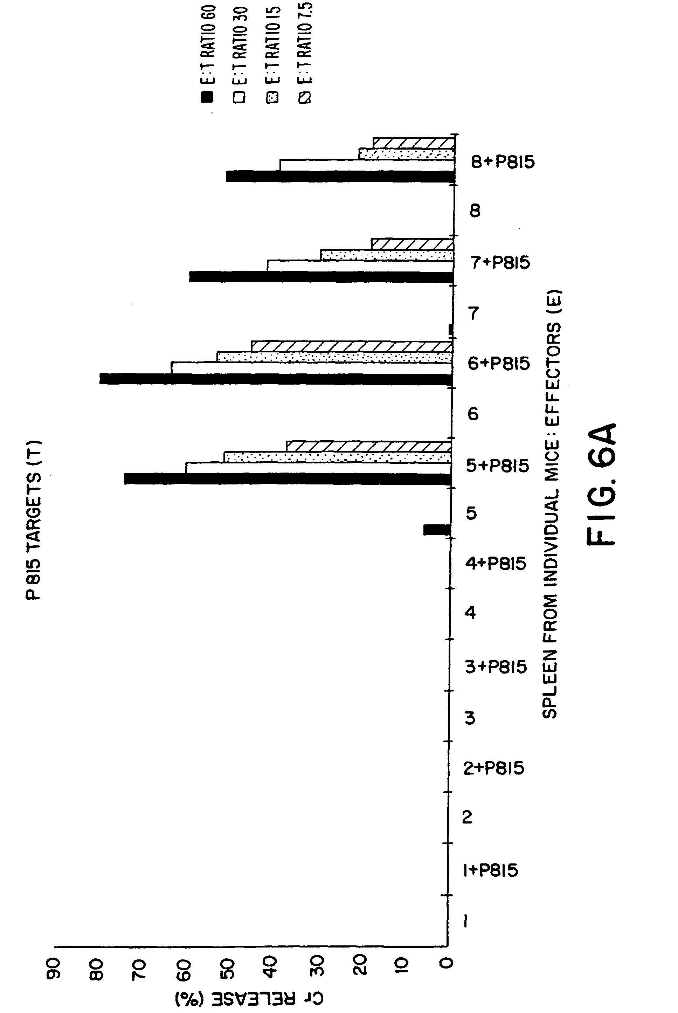

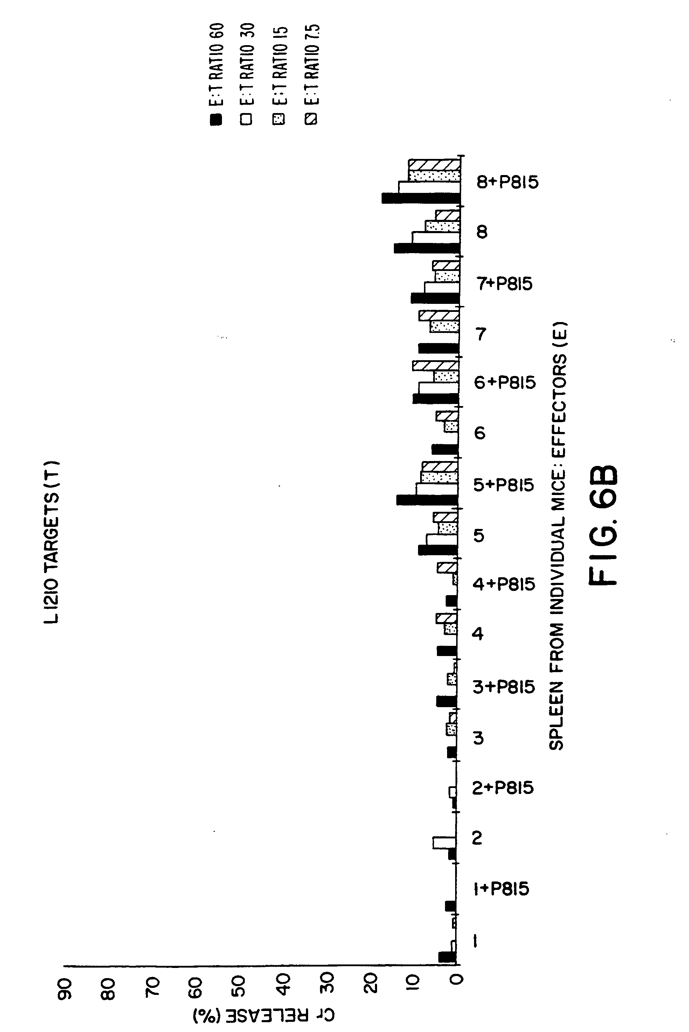

Figure 6. P 815 Targets (T). Chromium release assay on P815* and L1210 target cells with spleen cells from individual mice. Y-axis = Cr release (%). X-axis = spleen from individual mice:effectors (E). This figure shows that the spleen cells of the four P815*-resistant mice (see Fig. 5; individual mice nrs 5 - 8 in Fig. 6), contain a strong cytolytic activity directed against P815* cells (Fig. 6A) but not against the irrelevant (but MHC class I-matched) L1210 tumor cells (Fig. 6B). In contrast, the spleen cells of the four naive animals (mice nrs 1-4) do not show any detectable cytolytic activity against P815* cells (Fig. 6A). The spleen cells from individual mice (1 - 8) were cultured in vitro for five days either in the absence (x) or in the presence of P815* stimulator cells (x + P815). Thereafter, they were used as effector cells on chromium-labelled target cells, at different effector:target (E:T) ratios.

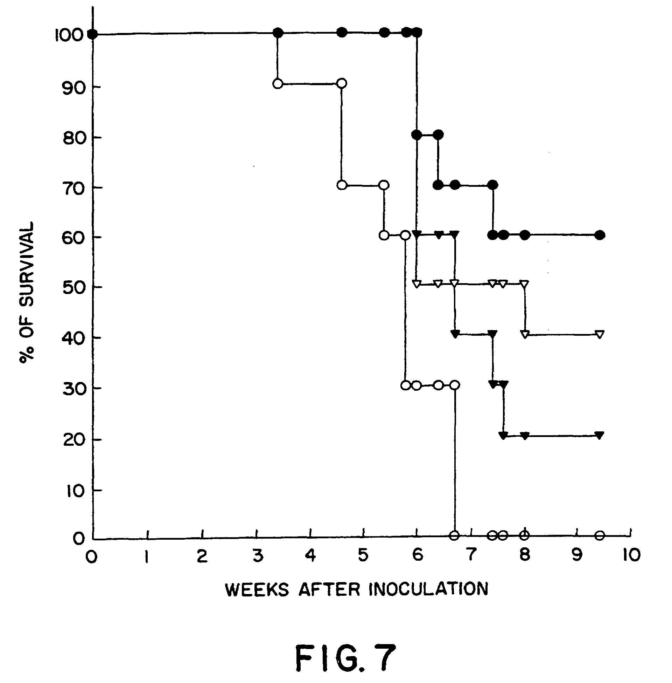

Figure 7. Survival of mice bearing an established tumor P815*. Y-axis = % of survival. X-axis = weeks after inoculation.
   - Key:: ○ untreated mice (n = 10)
   ● HY41 - treated mice (n = 10)
   ∇ HY62 - treated mice (n = 10)
   ▼ P815 - treated mice (n = 10)
   Survival curves of tumor-inoculated mice treated with irradiated HY41 or HY62 hybridoma cells. All mice were inoculated ip with 2 x 10⁵ P815* tumor cells on day 0. The figure shows that 2 months after tumor inoculation, 6/10 and 4/10 animals treated by 4 weekly ip injections of irradiated HY41 and HY62 hybridoma cells, respectively, were alive and tumor-free. In contrast, none (0/10) of the untreated animals and only 2/10 animals treated with irradiated P815* tumor cells were alive at that same time.

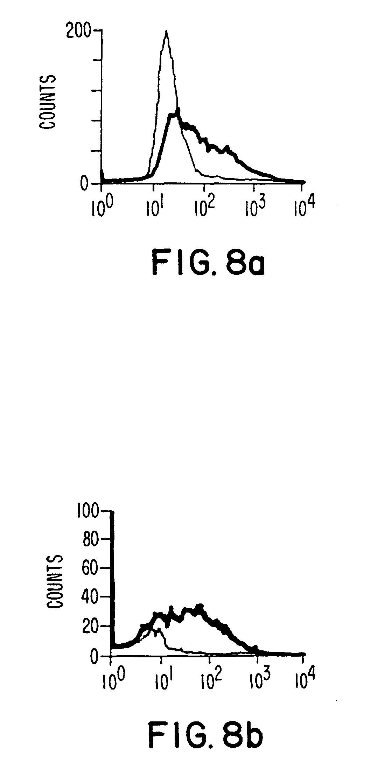

Figure 8. FACS analysis showing HLA-DR expression in human F3BG10 DLC/tumor cell hybridoma (Fig 8a) and in its subclone F3BG10-H12 (Fig 8b) before and after incubation with interferon γ. Before incubation with the cytokine, labeling by the anti-HLA-DR mAb (thinner line) was identical to the labeling by the isotope-matched control mAb (not shown). After 24 hours incubation with interferon γ, around 40% of the F3GG10 hybridoma cells and over 90% of the H12 subclone cells were specifically labeled by the anti-HLA-DR mAb (thicker line).

### Detailed Description of the Invention

The present invention relates to the use of DC/tumor cell hybrids and hybridomas for activating anti-tumor responses. Although the specific procedures and methods to produce said hybrids and hybridomas described herein are first exemplified using a DBA/2 mouse mastocytoma cell line and DLCs isolated from syngeneic spleen, they are merely illustrative for the practice of the invention. Analogous procedures and techniques are applicable for the treatment of human subjects, as thereafter exemplified using a human osteosarcoma cell line and blood-derived DLCs. Therefore, DC/tumor cell hybrids and hybridomas could be used to immunize human patients against their cancer. Procedures applicable to the treatment of a human subject would involve the following steps.
(1) A sample is provided of the tumor against which an immune response is needed. Such a sample can be obtained when the primary tumor and/or its metastases are removed by surgery, as practised for example for cancers of the breast, prostate, colon, and skin. When the treatment of the cancer involves chemotherapy and/or radiotherapy rather than surgery, as practised for example for small cell lung cancer, lymphomas and leukemias, a sample of the tumor can be obtained from a metastatic site, either before treatment or after relapse. Examples of easily-accessible tumor sampling sites are the peripheral blood, bone marrow, peritoneal and pleural effusions, lymph nodes and skin.
   Tumor cells can be separated from blood or bone marrow samples, for instance, by a combination of physical, enzymatic and immunological methods. Contaminating red blood cells can be removed by osmotic lysis. Tumor cells can be concentrated by density centrifugation. Tumor cells can be separated from other cells by binding antigen on the tumor cell surface to antibody-coupled magnetic beads, which are then separated from the biological fluid by means of magnets. In negative cell selection, which may be performed prior to positive cell selection, antibodies bind to antigens that are expressed on contaminating cells, and used to deplete the biological fluids of non-tumor cells. In positive cell selection, antibodies bind to tumor-associated antigens, and this binding is used to separate tumor cells from the biological fluids. When tumor cells are separated by means of antibody-coupled magnetic beads, cells can be released from the beads by digestion of the antigen/antibody binding sites with chymopapain or by other means. The resulting separated tumor cells can re-express the tumor-associated antigen after a short time in culture. The tumor cells are expected to contribute genes encoding known and unknown tumor-associated antigens to the hybridoma of the invention.
   Tumor cells can also be separated from solid tissue samples, using a combination of physical, enzymatic and immunological methods. Macroscopic peritumoral stromal tissue can be removed by dissection prior to reduction of the tumor to a cell suspension. Density centrifugations and antibody-mediated separations can then be performed on the cell suspension as described above.
(2) The purified tumor cells are then prepared for cell fusion. Three types of tumor partners can be prepared: (i) primary cultured tumor cells, (ii) immortal tumor cells, and (iii) drug-sensitive immortal tumor cells. Primary cultured tumor cells are purified tumor cells which have been cultured for a limited period of time in the presence of appropriate growth factors. Immortal tumor cells are permanent cell lines derived from these primary cultured tumor cells; such permanent cell lines can be obtained, for instance, after culturing the primary tumor cells for longer periods of time in the presence of appropriate growth factors, or by transducing the primary tumor cells with immortalizing genes. Finally, drug-sensitive immortal tumor cells are permanent cell lines derived from spontaneous mutants of immortal tumor cells; these mutants are selected by culturing the immortal tumor cells in the presence of an appropriate drug. These drug-sensitive immortal tumor cells die when they are exposed to the drug to which they are sensitive. For example, 6-thioguanine was used to select the murine P815* mastocytoma cell line described in Example 1, and 5-bromo-2'-deoxyuridine was used to select the human 143B osteoasarcoma cell line described in Example 7. Both cell lines die when cultured in HAT-containing medium, as described in Examples 3 and 9.
   (2a) As an alternative to step (2) a pre-established immortal human tumor cell line can be used, provided that at least one of the tumor-associated antigens from the patient's tumor cells are matched to these pre-established immortal tumor cells.
(3) A sample is provided with a source of DCs. Such samples include for example peripheral blood, cord blood, bone marrow, lymph or accessible lymph nodes; they may be taken from the patient or from a healthy, HLA-compatible donor. From there, two alternatives are available. Functionally-competent DCs can be purified directly from these samples, using various methods described in the literature. Alternatively, functionally-competent DCs can be purified after in vitro differentiation of the precursors contained in these samples, which can be done by culturing the latter in the presence of cytokines, as described hereunder.
(4) The DCs are prepared for cell fusion, in one of the 4 following ways. (1 °) Primary DCs purified directly from blood, lymph or other tissues are maintained in culture for no longer than 24 hours. (2°) Primary cultured DCs differentiated from blood, bone marrow or other tissues are cultured for at least 7 days in the presence of cytokines, as described for human blood DCs in Example 8 or as published by Sallusto and Lanzavecchia, 1994, J. Exp. Med. 179: 1109-111; Romani et al, 1994, J. Exp. Med. 180: 83-93; Mackensen et al, 1995, Blood, 86: 2699-2707. (3°) Immortal DCs can be derived from primary-cultured DCs, for example by adapting the method described by Paglia et al, 1993, J. Exp. Med. 178: 1893-1901. These authors immortalized neonatal mouse spleen DLCs by using a recombinant retrovirus. (4°) HAT-sensitive variants of these DC lines can thereafter be derived by standard culture techniques, to yield drug-sensitive immortal DCs.
(5) A tumor cell partner is then fused with a DC partner. From there, two alternatives are available, namely to separate or not to separate the fused cells by metabolic selection. After fusion, the treated cells include a plurality of DC/tumor cell hybrids, as well as unfused tumor cells and unfused DCs. If no selection is applied, fused cells as well as unfused cells are used for inducing an anti-tumor immunity in vivo and/or in vitro (see § 6 ). If a metabolic selection is applied, for example by plating the treated cells in HAT-medium, only the immortal, HAT-resistant hybrid cells survive (Example 9) and permanent cell lines hereafter termed DC/tumor cell hybridomas are developed from them.
(6) The DC/tumor cell hybridomas with therapeutic potential are then selected from all growing hybridomas. Their therapeutic potential is linked to the retention of pertinent DC characteristics and of pertinent tumor cell characteristics. Pertinent DC characteristics include DC morphology, DC surface markers, DC genetic markers and the capacity to activate immune cells in vitro. At least one of these DC characteristics may suffice to qualify hybridomas made of (drug-sensitive) immortal tumor cells and primary cultured DCs, since these hybridomas necessarily inherited immortality from the tumor parent. (1°) The selection may be based on the morphologic DC appearance of the hybridoma by scanning electron microscopy (SEM), as shown in Example 4A and Figure 1. Such an analysis can be performed on a minute sample of cells at a very early stage of hybridoma development, allowing the culture efforts to be focused on the dendritic-like hybridomas. (2°) In the absence of morphological DC characteristics, as in Example 10A, the expression of DC surface markers may be used to select hybridomas with therapeutic potential. If such DC surface markers, including namely T-cell activating molecules, are not expressed on resting hybridomas, they may nevertheless be induced by treatment with cytokines or other activating agents, as described in Example 10B. (3°) Genetic DC markers are further used to confirm or to exclude the contribution of a T-cell, B-cell or other cell type to the hybridoma, as in Examples 4C and 10C. HLA-DR gene typing can also be used to identify blood donor genes when the tumor cell and the DC are from distinct individuals, as in Example 10C.
   In DC/tumor cell hybridomas involving patient's related pre-established immortal tumor cells, it is necessary to select dendritic-like hybridomas that express in addition at least one of the patient's matched tumor-associated antigens. Standard immunocytochemistry can be performed on small samples of the hybridomas to identify such tumor-associated antigens as Her2/neu for breast cancer and carcinoembryonic antigen (CEA) for colon cancer. The hybridomas identified as potentially useful are amplified in culture for complete phenotypic characterization (chromosomes, genetic markers, cell surface markers and sub-cellular morphology) and for clinical use.
   Thirteen embodiments of our invention relating to the use of DC/tumor cell hybrids or hybridomas are briefly described as follows:
   Embodiments A, B, C: primary cultured patient's tumor cells are fused with primary cultured DCs purified from blood, lymph or other tissue (A), or with primary cultured DCs differentiated from precursors derived from blood, bone marrow or other tissue (B), or with immortal DCs (C), to yield a plurality of DC/tumor cell hybrids that are are used without selection.
   Embodiments D, E: primary cultured patient's tumor cells are fused with immortal DCs (embodiment D) or with drug-sensitive immortal DCs (embodiment E) to yield a plurality of DC/tumor cell hybridomas; the latter are mixed in embodiment D with unfused immortal DC. In these embodiments, hybridomas with both DC characteristics and tumor cell characteristics may be selected for further use.
   Embodiments F, G: patient's immortal tumor cells are fused with primary cultured DCs purified from blood, lymph or other tissue (F), or with primary cultured DCs differentiated from precursors (G), to yield a plurality of DC/tumor cell hybridomas, mixed with unfused immortal tumor cells. In these embodiments, hybridomas with DC characteristics are selected for further use.
   Embodiments H, I: patient's drug-sensitive immortal tumor cells are fused with primary cultured DCs purified from blood, lymph or other tissue (H), or with primary cultured DCs differentiated from precursors (I), to yield a plurality of DC/tumor cell hybridomas. In these embodiments, hybridomas with DC characteristics are selected for further use.
   Embodiments J, K: Patient's related, pre-established immortal tumor cells are fused with primary cultured DCs purified from blood, lymph or other tissue (J), or with primary cultured DCs differentiated from precursors (K), to yield a plurality of DC/tumor cell hybridomas, mixed with unfuscd immortal tumor cells. In these embodiments, hybridomas with DC characteristics and expressing in addition the patient's matched tumor-associated antigen(s) may be selected for further use.
   Embodiments L, M: Patient's related, pre-established, drug-sensitive immortal tumor cells are fused with primary cultured DCs purified from blood, lymph or other tissue (L), or with primary cultured DCs differentiated from precursors (M), to yield a plurality of DC/tumor cell hybridomas. In these embodiments, hybridomas with DC characteristics and expressing in addition the patient's matched tumor-associated antigen(s) may be selected for further use.
(7) The selected hybridomas are then used for inducing an anti-tumor immunity, either in vivo or in vitro, thereby contributing to the rejection of the residual tumor in the patient. For the induction of an anti-tumor immune response in vivo, the DC/tumor cell hybridomas are irradiated or otherwise inactivated, and injected, for example sub-cutaneously, into the patient. The patient is monitored for signs of an anti-tumor immune response and for the clinical evolution of his/her cancer. In a murine model, a single injection of a living DC/tumor cell hybridoma into syngeneic mice elicited an anti-tumor immune response. In addition, multiple injections of an irradiated DC/tumor cell hybridoma had a therapeutic effect on mice preinoculated with a lethal dose of tumor cells. For the induction of an anti-tumor immune response in vitro, the DLC/tumor cell hybridomas are irradiated or otherwise inactivated, and cultured with the immune cells of the patient. The activated immune cells are then re-injected into the patient. The patient is monitored for the presence of an anti-tumor immune response and for the clinical evolution of his/her cancer.

DCs can be dendritic cells of myeloid origin, monocytes, cells intermediate between dendritic cells and monocytes.

Herein, the term "DC/tumor cell hybrid" is defined as a fused cell which exhibits characteristics of both a DC and the specific tumor cell of interest. Since DC may be a dendritic cell, or a monocyte, DC/tumor cell hybrids may include hybrids with different phenotypic characteristics reflecting these different cell fusion partners. A plurality of DC/tumor cell hybrids is capable of eliciting an immune response, either in vivo or in vitro, against the tumor fusion partner which makes up part of the genome of the hybrids. This capacity is not inhibited by the presence of unfused DCs, DC lines or unfused tumor cells or tumor cell lines.

Herein, the term "DC/tumor cell hybridoma" is defined as an immortal hybrid cell line, which exhibits characteristics of both a DC and the specific tumor cell of interest. Since DC may be a dendritic cell, or a monocyte, DC/tumor cell hybridomas may exhibit phenotypic characteristics of any of these cell lines. For instance, in examples below, 1 human DC/tumor cell hybridoma was likely from monocytic origin. More importantly, a DC/tumor cell hybridoma is capable of eliciting an immune response, either in vivo or in vitro, against the tumor fusion partner which makes up part of the genome of the hybridoma.

Herein, the term "anti-tumor response in vivo" refers to the in vivo induction of immune effectors that confer resistance to a subsequent challenge with tumor cells, and contribute to the rejection of pre-existing tumor cells. In Example 5B, these immune effectors include cytotoxic T-lymphocytes that were detected by submitting the spleen cells of the immunized animals to an in vitro assay. In human subjects, appropriate non-invasive measures can be used for demonstrating the presence of anti-tumor immune effectors. However, the clinical course of the tumor, monitored by imaging techniques and the survival of the patient, will be the prime criterion for the evaluation of the immunotherapy.

Herein, the term "anti-tumor response in vitro" refers to the in vitro activation of autologous immune cells into anti-tumor immune effectors. The latter will contribute to the rejection of the pre-existing tumor cells when infused into the patient The secretion of IL-2 by the murine T-DLC/tumor cell hybridomas (Example 6) and the secretion of GM-CSF by the human (presumed monocytic) DC/tumor cell hybridoma may contribute to such in vitro and in vivo activation of anti-tumor immune cells.

Herein, the term "DC characteristics" shared by the hybridoma of the invention refers to DC morphology, the expression of DC surface markers, the expression of DC genetic markers and/or the activation of immune cells.

Herein, the term "DC morphology" refers to a typical image observed. by scanning electron microscopy. The images of the DC/tumor cell hybridoma are compared to those of the parent tumor cell and DC. At first glance, to one skilled in the art, it is clear that the hybridoma resembles the DC more than the tumor cell. Upon analysis, DLCs have irregular shapes, due to the presence of clearly-visible, flat cytoplasmic extensions like pseudopodia and veils. Hybridomas with such similar cytoplasmic extensions can be recognized as having a dendritic-like cell morphology, as illustrated in Fig. 1 (see Example 4). These data are also consistent with the possibility that other embodiments of the present invention may express these or other DC morphological traits, since the DC morphology of a DC/tumor cell hybridoma is expected to mirror the particular morphology of the DC used as a fusion partner.

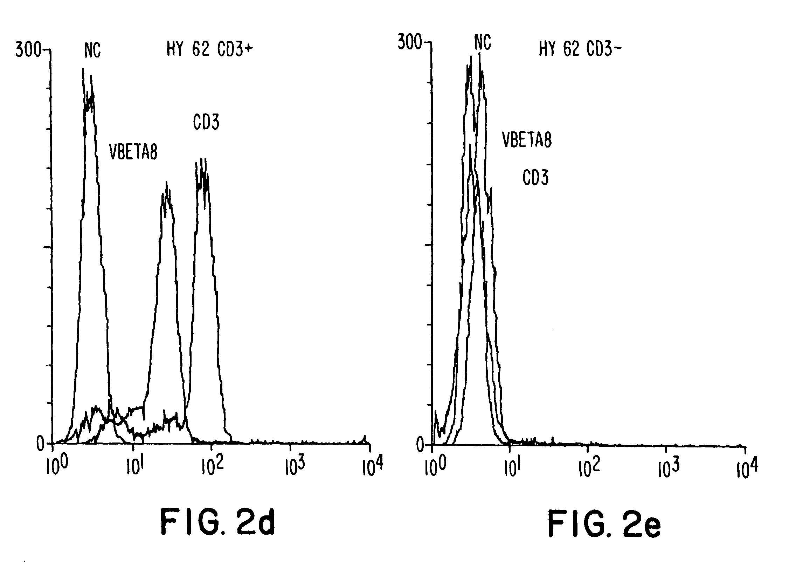

Herein, the term "expression of DC surface markers" refers to the expression of markers restricted to the DCs used for fusion. These markers include T-cell activating molecules and other molecules. T-cell activating molecules are expressed on activated APCs; they include mainly MHC class I and class II restricting elements, as well as the family of B7 costimulatory molecules, the latter bind to the CD28/CTLA4 counter-receptor on T-cells. Other DC surface markers include, for example, CD1a for human myeloid dendritic cells, and CD14 for monocytes. It is shown in Example 4B (Table 1 and Figure 2) that the HY41 and HY62 hybridomas express MHC class I molecules and the TCR/CD3 complex, but neither MHC class II molecules, nor B7 costimulators. When such T-cell activating molecules are not expressed on resting hybridomas, they can sometimes be induced by exposure to cytokines or other activating agents; Example 10B illustrates such an induced expression of HLA-DR on a human DC/tumor cell hybridoma.

Herein, the term "tumor-associated antigen" refers to a peptide derived from a protein expressed by a tumor cell which, when expressed by the hybridoma of the invention, will enable the hybridoma to elicit a tumor-specific response in vivo and/or in vitro . It also refers, by extension, to the proteins from which the antigenic peptides are derived, and to the genes encoding the antigenic proteins.

Herein, the term "activation of immune cells in vivo" refers to the immune rejection of a residual tumor, as measured by its reduction in size and by the survival of the patient, as shown for mice in Example 5C. In vitro correlates of this in vivo state of immunity include for example the detection of blood or tissue immune cells able to kill the patient's own tumor cells in vitro. In experimental animals, the quoted expression also refers to the immune rejection of the living hybridoma, to the immune resistance to a subsequent inoculation of tumor cells, and to the presence of tumor-specific cytolytic effector cells in the lymphoid organs of the tumor-resistant animals, as shown in Example 5.

Herein, the term "activation of immune cells in vitro" refers for example to a mixed lymphocyte-tumor cell reaction, wherein the dendritic cell/tumor cell hybridoma ("the tumor cell") stimulates one of the following reactions by allogeneic T-cells ("the lymphocyte"): (1) T-cell proliferation, as measured by tritiated thymidine incorporation; (2) T-cell secretion of cytokines including for example IL-2, interferon-gamma and others, as measured by ELISA, bioassay, or reverse transcription polymerase chain reaction; (3) T-cell-mediated tumor cell lysis, as measured by chromium release assay. This term may also refer to the activation of other immune cells, like monocytes and natural killer cells, and can be measured, for example, by cytokine release or cytotoxic cell assays.

The following experimental examples are provided to illustrate the invention.

### Example 1

### Preparation of Murine Tumor-Derived Cells.

The P815-X2 cell line was derived from the methylcholanthrene-induced mastocytoma P815 of mouse DBA/2 origin (Dunn and Potter, 1957, J. Natl. Cancer Inst. 18: 587-601. This cell line was obtained by Thierry Boon, director of the Ludwig Institute for Cancer Research, Brussels Branch, Belgium, and recloned by his group (Uyttenhove et al, 1980, J. Exp. Med 1562: 1175-1183). The subclone P1 was extensively used by T. Boon's group and given to the present inventors in 1980. A 6-thioguanine-resistant mutant was derived from P1, as described by Le et al, 1982, Proc. Natl. Acad. Sci. USA 79:7857-7861. Briefly, P1 cells were cultured in Dulbecco's modified Eagle's medium (Grand Island Biological Co., Grand Island, N.Y.) supplemented with 10% fetal calf serum (FCS) (Gibco BRL, Merelbeke, Belgium), in a 7% CO2 atmosphere. Increasing concentrations of 6-thioguanine (Sigma, Bornem, Belgium), ranging from 1 µg/ml to 30 µg/ml were added to the culture. The final 6-thioguanine-resistant cells died in HAT-medium, i.e. in medium supplemented with 10⁻⁴ M hypoxanthine, 3.8 x 10⁻⁷ M aminopterin, and 1.6 x 10⁻⁵ M 2-deoxythymidine (HAT supplement, Gibco BRL). Several HAT-sensitive clones were isolated by limiting dilution from these 6-thioguanine-resistant cells. A HAT-sensitive clone expressing MHC class I antigens was used in the present invention and will hereafter be called P815*.

P815* cells were cultured at 37°C in a 7% CO2 atmosphere in tissue culture flasks (Becton Dickinson, CA) containing RPMI 1640 medium (Seromed Biochem KG, Berlin, Germany) with 10% FCS (Gibco BRL). One day before use, P815* cells were diluted with fresh medium in order to be in exponential growth phase at the time of cell fusion.

### Example 2

### Preparation of Murine Dendritic-Like Cells from the Spleen

The preparation of splenic DLCs was done according to a multi-step procedure initially described by Crowley et al, 1989, Cell. Immunol. 118: 108-125. This procedure was adapted as described by Somasse et al, 1992, J. Exp. Med 175:15-21. The procedure was started one day before the fusion experiment and yielded 200,000 to 500,000 DLCs per spleen.

Briefly, DBA/2 mice were obtained from Charles River, Sulzfeld, Germany, and maintained in specific pathogen-free conditions. Animals 8 to 10 weeks old were killed by cervical dislocation; their spleens were quickly removed and kept in cold RPMI 1640 medium. The spleens were digested with collagenase (CLSIII; Worthington Biochemical Corp., Freehold, NJ) and separated into low and high density fractions on a bovine serum albumin gradient (Bovuminar Cohn fraction V powder; Armour Pharmaceutical Co., Tarrytown, NJ). Low-density cells were cultured during 2 hours in RPMI 1640 medium with 10% FCS, and the non-adherent cells were removed by vigorous pipetting. The latter were further cultured for 1 hour in serum-free RPMI 1640 medium. The non-adherent cells were removed by gentle pipetting and cultured overnight in RPMI 1640 medium with 10% FCS. The final non-adherent fraction contained at least 95% dendritic cells, as assessed by morphology and specific staining.

### Example 3

### Fusion of Murine Tumor Cells and Dendritic-Like Cells

The procedure used to fuse HAT-sensitive tumor cells with mortal splenic DLCs was adapted from procedures used in our laboratory to generate monoclonal antibodies, as described by Franssen et al, Protides of the Biological Fluids, editor H. Peeters, Pergamon Press, Oxford, 1982, pp 645-648.

Briefly, splenic DLCs and P815* cells were extensively washed in serum-free RPMI 1640 medium. Five million DLCs were mixed with the same number of HAT-sensitive P815* cells in a 15 ml conical tube and centrifuged. Two hundred µl of a 50% solution of polyethylene glycol (PEG 4000, Merck AG, Darmstadt, Germany) in RPMI 1640 medium were added dropwise to the cell pellet. The fusion was then stopped by the stepwise addition of RPMI 1640 medium.

The cells were washed to remove the PEG and resuspended in RPMI 1640 medium with 10% FCS. After 2 hours incubation at 37°C, the cells were centrifuged, resuspended in RPMI 1640 medium containing HAT and 10% FCS, and plated at 10⁴ cells/well in flat-bottomed 96-well plates (Becton Dickinson, CA). The plates were seeded one day before use with a feeder layer consisting of 5,000 irradiated peritoneal cells/well. Peritoneal cells were taken from Balb/c mice and irradiated at 2,000 rads from a Cobalt 60 source before plating. The plated fusion was cultured at 37°C in a 7% CO2 atmosphere. The medium (RPMI 1640 with 10% FCS and HAT) was renewed as required by cell growth. In these conditions, unfused DLCs, that are not immortal, died within a few days of culture; unfused P815* cells, that are immortal but HAT-sensitive, died in the HAT-containing-medium, and only hybrid cells, combining the immortality of P815* cells with the HAT-resistance of DLCs survived and developed into growing DLC hybridomas.

After 3-4 weeks of culture, wells that contained a growing DLC hybridoma could be clearly identified by phase contrast microscopy. The content of a positive well was transferred into a larger well (24-well plates, Becton Dickinson, CA) previously seeded with irradiated peritoneal cells. Eventually, DLC hybridomas were transferred to small tissue culture flasks (Becton Dickinson, CA) and amplified for characterization and storage in liquid nitrogen.

### Example 4:

### Selection of Murine Dendritic-Like Cell/Tumor Cell Hybridomas with Therapeutic Potential

The goal of these experiments was to select DLC/tumor cell hybridomas exhibiting at least one of the three following characteristics: (1°) a DLC morphology; (2°) DLC surface markers; (3°) DLC genetic markers.

### A. Dendritic-Like-Cell Morphology

P815* tumor cells, fresh splenic DLCs, and DLC/tumor cell hybridomas were analyzed by scanning electron microscopy (SEM). About one million cells were fixed in 2-4% glutaraldehyde for 24 hours at room temperature and washed in phosphate buffer saline. Cell suspensions were then collected on 0.2µM nylon filters, postfixed in 1% osmium tetroxide followed by 1% tannic acid mordant and uranyl acetate, with a series of saline washes in between each step. The samples were dehydrated through graded alcohols, then critical point dried from CO2. After critical point drying, the samples were mounted on aluminium stubs and sputter coated with gold using a Bio-Rad PS3 coating unit. The cells were examined at 20 kV in a Hitachi S520 scanning electron microscope.

Photographs of the cells are shown in Fig. 1. In these conditions, P815* tumor cells appeared as uniform rounded cells, whose surface was spiked with numerous short microvilli (Fig. 1a). In contrast, splenic DLCs appeared as irregular cells, due to the presence of clearly-visible cytoplasmic extensions, resembling pseudopodia and veils. Furthermore, the DLC surface was not spiked with numerous microvilli, but displayed instead fewer, larger protrusions. The hybridoma cells were in general much larger than the parent P815* cells. Many of them (like the one named HY1) looked very much like the P815* parent, which was linked to their round regular shape and microvilli-like protrusions (Fig. 1c). In contrast, hybridomas HY41 and HY62 looked much more like the DLC parent, when considering their irregular shape and relatively bare cell surface with some large protrusions, as shown for HY41 in Fig. 1d. However, a DLC morphology may be assumed not only by dendritic cells of myeloid origin, but also by cells derived from other lineages, including cells of the B- and T- lymphocyte lineages, like follicular dendritic cells and dendritic epidermal T-cells, respectively. In order to determine the cell lineage of the DLC that fused with the P815* tumor cell, other DLC characteristics were investigated for hybridomas HY41 and HY62.

### B. Dendritic-Like-Cell Surface Markers

Cell surface molecules were characterized by FACS analysis, as described by Flamand et al, 1990, J. Immunol 144:2875-2882. Briefly, the cells were preincubated with 2.4G2, a rat anti-mouse Fc-receptor (Fc-R) monoclonal antibody (mAb) for 10 min prior to staining with fluorescein-coupled monoclonoal antibody (fl. mAb). This preincubation was done to prevent the non-specific binding of mAb to cellular Fc-R. When unlabelled mAb were used, they were revealed by incubation with fluoresceinated anti-IgG antibodies. The labelled cells were gated for size and side scatter to eliminate dead cells and debris, and analyzed on a Facscan (Becton Dickinson, CA).

The results are summarized in Table 1. No T-cell activating molecules or other dendritic-cell-associated molecules were expressed by the HY41 and HY62 hybridomas. However, a fraction of the cells of both hybridomas expressed surface CD3ε chains of the T-cell receptor (TCR), suggesting that they were T-lymphocyte/tumor cell hybridomas. After cloning by limiting dilution, CD3+ and CD3-subclones were isolated from both hybridomas. Figure 2 shows that the HY41 and HY62 CD3ε+ subclones were also labeled by a fl mAb specific for the V β8 domain of the TCR, whereas P815* tumor cells and the CD3ε-subclones remained unstained. These results showed that the HY41 and HY62 hybridomas expressed an α/b TCR, and hence had incorporated a dendritic-like T-lymphocyte. However, neither CD4 or CD8 were expressed by the hybridomas. In order to confirm these cell surface marker studies, genetic marker studies were undertaken.

**Table 1.**

| Cell Surface Markers of Murine Dendritic-Like-Cell/Tumor Cell Hybridomas HY41, HY62 and Parent Cells | | | | | |
|---|---|---|---|---|---|
| Reagents | Surface markers | DLCs¹ | P815* | HY41 | HY62 |
| | Present on DLCs and P815* | | | | |
| 31.3.4 mAb | MHC class I Kd | + | + | + | + |
| 34.4.20 mAb | MHC class I Dd | + | + | + | + |
| 30.5.7 mAb | MHC class I Ld | + | + | + | + |
| 3E2 fl mAb | ICAM-1 (CD54) | + | + | - | - |
| | | | | | |

| | Present on P815* only | | | | |
|---|---|---|---|---|---|
| 2.4G2 mAb | Fc-R | - | + | - | - |
| | | | | | |

| | Present on DLCs only | | | | |
|---|---|---|---|---|---|
| | T-cell activating molecules: | | | | |
| 14.4.4 fl.mAb | MHC class II | + | - | - | - |
| 16-10A1 fl mAb | B7-1 (CD80) | + | - | - | - |
| GL1 fl mAb | B7-2 (CD86) | + | - | - | - |
| CTLA4- human Ig | CTLA4-ligand | + | - | - | - |
| M1/69 fl mAb | HSA (CD24) | + | - | - | - |
| | | | | | |
| | Other molecules: | | | | |
| N418 fl mAb | N418 (CD11c) | + | - | - | - |
| 145-2 C11 | CD3ε | nd² | - | + | + |
| F23-1 | TCR V β8 chain | nd | - | + | + |
| H129.19 | CD4 | nd | - | - | - |
| 53-6.7 | CD8a | nd | - | - | - |

| | | | | | |
|---|---|---|---|---|---|
| ¹: By cell scatter and cell surface marker analyses, DLCs contained more than 95% dendritic cells; | | | | | |
| ²: nd: not detectable 31.3.4, 34.4.20, 30.5.7: mouse anti-mouse H2-K^{d}, D^{d} and L^{d} mAb, respectively; Ozato et al, 1980, J. Immunol. 124:533-; 3E2: hamster anti-ICAM-1, from Pharmingen, San Diego, CA 2.4G2 : rat anti-mouse Fc-gamma-RII/III mAb (Unkeless, 1979, J. Exp Med 150:580-586; 14.4.44: mouse anti-I-E^{d} fluorescein-coupled mAb (fl mAb);Ozato et al, 1980, J. Immunol. 124:533-16-10A1: rat anti-B7-1 fl mAb; Razi-Wolf et al, 1993, Proc. Natl. Acad. Sci. USA 90:11182-11186; GL1: hamster antiB7-2 fl mAb;Hathcock et al, 1993, Science 262:905-907; CTLA4-human IgG fusion protein: Linsey et al, 1991, J. Exp. Med 174:561-569; M1/69: Rat anti-HSA, from Pharmingen, San Diego, CA. N418: hamster anti-mouse CD11c; Metlay et al, 1990, J. Exp. Med 171:1753-1771; 145-2C11: hamster anti-mouse DC3ε fl mAb; Leo et al, 1987, Proc.Natl.Acad.Sci.USA 84:1374 F23.1: mouse anti-mouse TCR V β8 fl mAb from ATCC, Bethesda MD. H129.19: rat anti-mouse CD4 fl mAb, from Gibco BRL, Gaithersburg, MD. 53-6.7: rat anti-mouse CD8a fl mAb, from Gibco BRL, Gaithersburg, MD. ND: not detectable | | | | | |

### C. DLC Genetic Markers

First, Southern blot analysis was used to analyse the rearrangement status of the TCR genes in genomic DNA from the HY41 hybridoma. The mouse T-cell hybridoma 13.26.8-H6 was used as a reference for rearranged TCR genes (Ruberti et al, 1992, J. Exp. Med. 175: 157-162), and P815* mastocytoma cells as well as DBA/2 spleen cells were taken as controls for germ line TCR genes. Genomic DNA was extracted from 2 x 10⁷ cultured cells and from spleens, using the Genome DNA Kit (Bio 101, CA, USA) according to the manufacturer's instructions. Ten µg of DNA were digested for ± 4 hours with various restriction enzymes, separated on a 1% agarose gel and transferred to a nylon membrane (Qiabrane Nylon plus, Qiagen, Hilden, Germany) according to standard procedures. The blot was hybridized to a DIG-labeled synthetic oligonucleotide of 50 bases targeted to the first exon of the constant region of the mouse TCR β chain and processed for chemiluminescent detection using Boehringer Mannheim's DIG detection kit. The results showed that the HY41 genome contained a rearranged TCR β chain gene, which is a hallmark of T-cell lineage commitment (not shown).

Next, the Polymerase Chain Reaction (PCR) was used to detect rearranged V β8-Cβ sequences of the TCR in genomic DNA. The upstream primer was targeted to bases 47-66 with respect to the ATG initiation codon of the mouse V β8 region (5'-AACACATGGAGGCTGCAGTC-3') and the downstream primer was targeted to bases 141-160 of the fisrt exon of the Cβ region (5'-GTGGACCT CCTTGCCATTCA-3'). The PCR was carried out essentially according to the instructions of Boehringer Mannheim's Long Range Expand PCR System. Analysis of the PCR products on a 1% agarose gel stained with ethidium bromide is shown in Figure 3. A fragment with the expected length (4.5 to 5 kb)of the rearranged Vβ8-Cβ fragment is clearly seen in DNA from the T-cell hybridoma 13-26-8-H6 (lane T), used as a positive control, as well as in DNA from the HY41 and HY62 hybridomas (lanes 41 and 62); this fragment is not amplified in DNA from P815* tumor cells and from spleen cells (lanes P and S), used as negative controls. These results confirm that the DLC that fused with a P815* tumor cell to yield the HY41 and HY62 hybridomas was a T-lymphocyte expressing an α/β TCR receptor, including the Vβ8 domain. These hybridomas will hereafter be termed T-DLC/tumor cell hybridomas.

In conclusion, the HY41 and HY62 T-DLC/tumor cell hybridomas were selected for further studies because of their DLC morphology and T-lymphocyte lineage. In both hybridomas, the T-lymphocyte fusion partner was a rare and undetectable contaminant of the splenic DLC preparation. In view of the complex genetic regulations controling CD4 and CD8 expression in somatic cell hybrids (Wilkinson et al, 1991, J. Exp. Med. 174: 269-280), it is impossible to determine a posteriori if the fusing T-cell was a CD4+, CD8+, or CD4-CD8- "double negative" T-cell. However, whatever the sublineage of T-lymphocyte involved, the next step was to determine the in vivo immunogenicity of these T-DLC/tumor cell hybridomas.

### Example 5

### In vivo Immunogenicity of Murine T-Dendritic-Like-Cell/Tumor Cell Hybridomas

The goal of these experiments was to determine if the hybridomas induced an efficient immune rejection in vivo, as measured by the following criteria: (1°) rejection of the hybridomas by immunocompetent mice; (2°) vaccination with the hybridomas against a subsequent inoculation of tumor cells; (3°) treatment with the hybridomas after prior inoculation of tumor cells.

### A. Immune Rejection of T-DLC/Tumor Cell Hybridomas

Groups of 10 to 12 DBA/2 mice were injected intra-peritoneally with 500,000 living cells of the P815* tumor or of the HY41 hybridoma. Injected animals included mice immunosuppressed by sub-lethal irradiation as well as immunocompetent mice. All irradiated animals died from their tumor within four weeks of inoculation, showing that the HY41 and P815* cell lines were very similar in their tumorigenicity (Fig. 4). In contrast, 9/12 (75%) immunocompetent animals injected with the HY41 hybridoma survived two months after inoculation, when only 2/10 (20%) mice had survived the parental tumor injection. This experiment showed that the HY41 hybridoma was as tumorigenic as the parent tumor in irradiated mice, but more immunogenic than P815* in immunocompetent mice. Similar results were obtained with hybridoma HY62 (not shown).

### B. Induction of Tumor Resistance by Murine T-DLC /Tumor Cell Hybridomas

The 9 surviving HY41-treated mice, as well as 9 untreated animals, were challenged intra-peritoneally with 500,000 P815* cells. All (9/9) untreated mice died from their tumor within six weeks of inoculation, showing that the tumor cell injection was lethal for unimmunized animals. By contrast, 7/9 HY41-treated animals were still alive 6 weeks after tumor challenge, and 4/9 of them survived for at least three months (Fig. 5). These results strongly suggested that prior treatment of syngeneic mice with living HY41 DC hybridoma cells induced a memory immune response against the parent P815* cell line, conferring tumor resistance to 44% of the treated animals. A similar tumor-resistance could be induced by the injection of living HY62 hybridoma cells (not shown).

The spleens of the 4 P815*-resistant mice were tested in vitro for the presence of anti-P815* cytotoxic T-cells, as described by Moser et al, 1987, J. Immunol 138: 1355-1362. Briefly, spleen cell suspensions were stimulated in vitro during 5 days with the irradiated P815* cells, in order to induce a measurable memory response. They were then used as effector cells on chromium-loaded P815* and L1210 target cells. The latter have the same MHC class I haplotype (H-2d) as P815 cells. At several effector/target ratios, the spleen cells of the untreated animals completely failed to lyse the P815* and the L1210 target cells (Figs 6A and 6B). In contrast, the spleen cells from the 4 P815*-resistant mice lysed efficiently and specifically the P815* targets, without showing any significant activity on the L1210 targets. These results showed that the HY41-treated, P815*-resistant animals were able to mount a strong and tumor-specific cytolytic response upon in vitro restimulation.

### C. Induction of Tumor Treatment by Murine T-DLC/Tumor Cell Hybridomas.

In this experiment, 40 DBA/2 mice received an ip injection of 2 x 10⁵ P815* tumor cells. Seven days later, the mice were divided into 4 groups of 10 animals; the first group was left untreated while the 3 other groups were treated by 4 weekly ip injections of 2 x 10⁶ irradiated (15,000 F) P815*, HY41 or HY62 cells. The data are presented in figure 7. Untreated animals all died within 7 weeks of tumor inoculation, and 20% of the mice treated with irradiated P815* tumor cells survived, confirming the weak immunogenicity of the P815 tumor cell line. In contrast, 60% and 40% of the mice treated with irradiated HY41 and HY62 hybridoma cells, respectively, survived the prior injection of a lethal dose of P815* cells. These data showed that hybridoma HY41, and to a lesser extent hybridoma HY62, could induce the immune rejection of an established tumor. However, the mechanism leading to such an efficient in vivo immune rejection remained unclear. One possibility that was explored concerned the secretion of immunomodulating cytokines.

### Example 6:

### In vitro analysis of cytokine expression by T-DLC/tumor cell hybridomas

The goal of these experiments was to determine whether the HY41 and HY62 hybridomas synthesized some cytokines that could account, at least in part, for their in vivo immunogenicity. Total RNA was prepared from activated spleen cells, from P815* tumor cells and from the HY41 and HY62 hybridomas according to standard procedures. The Reverse-Transcription Polymerase Chain Reaction (RT-PCR) and cytokine-specific primers were used to amplify IL-2, IL-4, IL-10 and interferon γ (IFNγ) mRNA sequences, as described by De Wit et al, J. Immunology, 1993, 150: 361-366. The primers used to amplify IL-12 p40 sequences were 5'-TTCAACATCAAGAGCAG TAGC-3' and 5'-GGAGAAGTAGGAATGGGGAGT-3'. Analysis of the RT-PCR products on ethidium bromide-stained agarose gels showed that P815* tumor cells constitutively expressed IL-4 mRNA and that the HY41 and HY62 hybridomas constitutively expressed IL-2 and IL-4 mRNAs, but not IL-10, IL-12, and IFNγ mRNAs. These cytokine mRNAs were neverthesless detected in activated spleen cells, used as a positive control. In conclusion, these data showed that the HY41 and HY62 T-DLC/tumor cell hybridomas constitutively expressed IL-4 like the parent P815* tumor cell, and IL-2, like the parent T-lymphocyte. These cytokines, if secreted in vivo, may at least partially contribute to the immunogenicity of the hybridomas.

### Example 7

### Preparation of Human Tumor-Derived Cells

The human 143B thymidine kinase negative osteosarcoma cell line (hereafter termed 143B) is a HAT-sensitive cell line that was purchased from the ATCC (CRL n° 8303). The cells were cultured in Dulbecco's modified Eagle's medium supplemented with 10% FCS, 2% penicillin/streptomycin, 1% sodium pyruvate (all from Gibco BRL, Merelbeke, Belgium) and 0.015 mg/ml of 5-bromo-2'-deoxyuridine (Sigma Chemical Co, St Louis, MO). One day before fusion, the cells were diluted with fresh medium in order to be in exponential growth phase.

### Example 8

### Preparation of human Dendritic-Like Cells from Peripheral Blood.

Dendritic cells were differentiated in vitro from adherent blood precursors, using an adaptation of the technique described by Romani et al, 1994, J. Exp Med. 180: 83-93. Briefly, peripheral blood mononuclear cells (PBMC) were isolated from the buffy coat of a healthy donor by density gradient centrifugation on lymphoprep (Gibco BRL). Adherent cells were prepared by plating 10⁷ PBMC on 6-well tissue culture plates in 3 ml RPMI supplemented with 200 mM L-Glutamine, 50 µM Mercaptoethanol and 10% FCS. After 2 hours incubation at 37°C, the non-adherent cells were discarded by a very gentle rinse, and the adherent cells were further cultured in the above-described medium supplemented with GM-CSF (Leucomax, 800 U/ml) and IL-4 (Genzyme, 500 U/ml), at 37°C in a humidified atmosphere with 5% CO₂. After 7 days of culture, DLCs were recovered and characterized by cell scatter and cell surface marker analysis. The DLCs used for fusion contained 50% of monocytic-like cells, expressing CD14 but not CD1a or CD1c, as well as 38% of T lymphocytes, 4% of NK cells and 8% of B lymphocytes.

### Example 9

### Fusion of Human Tumor Cells and Dendritic-Like Cells

The procedure used to fuse HAT-sensitive tumor cells with DLCs was adapted from procedures used to generate monoclonal antibodies (Current Protocols in Immunology, chapter 2.5.4). The 143B tumor cells and the DLCs were extensively washed in serum-free medium (RPMI 1640); 2 x 10⁶ DLCs were mixed with 1 x 10⁶ 143B osteosarcoma cells and centrifuged. The pellet was resuspended in 500 µl of a 50% solution of polyethylene glycol (PEG 4000, Gibco) in Dulbecco's phosphate buffered saline without Ca⁺⁺, Mg⁺⁺ (ref. 14030035). After 1 minute, the PEG was progressively diluted by the slow and progressive addition of serum-free medium. The cells were washed free of PEG and resuspended in RPMI 1640 with 10% FCS. They were eventually plated at 2 x 10⁴ cells/well in flat-bottomed 96-well plates (Falcon, Becton Dickinson) and cultured in a 5% CO₂ atmosphere at 37°C. HAT medium was added to the wells 24 hours after fusion and renewed every two days. In these conditions, unfused DLCs died within 2-3 weeks of culture, unfused 143B osteosarcoma cells died in HAT-medium and only hybrid cells combining the immortality of the tumor cell with the HAT-resistance of a DLC survived and developed into growing cell lines. After 3-4 weeks of culture, wells containing growing cell lines were clearly identified by phase contrast microscopy. Their contents were transferred into larger wells and eventually into culture flasks for amplification. Culture stocks were frozen in liquid nitrogen before analysis.

### Example 10

### Identification of Human Dendritic-Like Cells/Tumor Cell Hybridomas with Therapeutic Potential

The goal of these experiments was to identify human DLC/tumor cell hybridomas presenting at least one of the three following characteristics: (A) DLC morphology; (B) DLC surface markers; (C) DLC genetic markers.

### A. DLC morphology

The 143B osteosarcoma cells and a series of hybridoma cells were anaiyzed by SEM, as described in example 4. Comparison of the parent cells and hybridoma cells showed that none of the hybridomas analysed, including F3BG10 cells, displayed morphologic dendritic-like features. In the absence of such features, other dendritic-like features were analyzed, namely the presence of DLC surface markers.

### B. DLC surface markers

Cell surface markers were analysed as described in Example 4. Results are summarized in Table 2. None of the tested hybridomas, including F3BG10 cells, expressed the T-cell activating molecules HLA-DR, B7.1, and B7.2. However, they expressed HLA class I, ICAM-1 (CD54) and LFA-3 (CD58), which were also present on the 143B tumor cells. They failed to express typical dendritic-cell markers like CD1a and CD1c, as well as markers specific for T-cells (CD3), B-cells (CD19), NK cells (CD56) and monocytes (CD14).

Since the hybridomas tested failed to express constitutively T-cell activating molecules, they were stimulated with a variety of cytokines in order to induce such expression. It was found that 40% of the F3BG10 hybridoma cells were induced to express varying amounts of surface HLA-DR after a 24 hour incubation with interferon γ. After cloning by limiting dilution, subclones were tested for their capacity to express induced HLA-DR. Figure 8 shows that at least 90% of H12 cells clearly expressed induced HLA-DR, which greatly increases their immunogenic potential.

**Table 2.**

| Cell Surface Markers of Human Dendritic-Like-Cell/Tumor Cell Hybridoma F3BG10 and Parent Cells | | | | |
|---|---|---|---|---|
| Reagents from | Surface markers | DLCs¹ | 143B | F3BG10 |
| | Present on DLCs and 143B | | | |
| Pharmingen | HLA class I | + | + | + |
| Immunotech | ICAM-1 (CD54) | + | + | + |
| Becton Dickinson | LFA-3 (CD58) | + | + | + |
| | | | | |

| | Present on DLCs only | | | |
|---|---|---|---|---|
| | T-cell activating molecules: | | | |
| Becton Dickinson | HLA-DR | + | - | -² |
| Innogenetics | B7.1 (CD80) | + | - | - |
| Pharmingen | B7.2 (CD86) | + | - | - |
| | | | | |
| | Other molecules: | | | |
| Immunotech | CD1a | + | - | - |
| Immunotech | CD1c | + | - | - |
| Becton Dickinson | CD14 | + | - | - |
| Becton Dickinson | CD2 | + | - | - |
| Becton Dickinson | CD3 | + | - | - |
| Becton Dickinson | CD19 | + | - | - |

| | | | | |
|---|---|---|---|---|
| ¹: By cell scatter and cell surface marker analyses, DLCs contained 50% monocytes, 38.% T-lymphocytes and 4% NK cells; the suspension also contained 8% of B-lymphocytes. | | | | |
| ²: HLA-DR expression could be induced in 40% of F3BG10 cells and in 90% of its H12 subclone by incubation with interferon γ. | | | | |

### C. DLC genetic markers

The goal of this first experiment was to determine whether the F3BG10 hybridoma had been generated by the fusion of a DLC with the 143B tumor cell, and to exclude that it was a revertant 143B tumor cell clone, that had become resistant to HAT-medium by mutation. This was done by typing the HLA-DR genes of the blood donor, of the 143B tumor cell and of the F3BG10 hybridoma. Genomic DNA was prepared according to standard procedures from 143B tumor cells, from the PBMC of the blood donor and from F3BG10 hybridoma cells. These DNAs were submitted to a non-isotypic HLA-DR B oligotyping method, described for the typing of DR B 1, 3, 4, 5 alleles by Buyse et al. 1993, Tissue Antigens 41: 1-4. The polymorphic second exon of the corresponding genes was amplified by PCR, and biotinylated nucleotides were incorporated into the amplifying fragments during this procedure. The PCR products were hybridized with a combination of 31 sequence-specific oligonucleotide probes, immobilized in parallel lines on membrane strips. After a stringent wash, streptavidin-labelled alkaline phosphatase was added to mark the biotinylated DNA fragments. The addition of the BCIP/NBT chromogen resulted in a colored precipitate. All reagents were part of the Innolipa DRB Key kit purchased from Innogenetics (Zwijndrecht, Belgium). The F3BG10 lane showed a mixture of bands corresponding to alleles present in the 143B osteosarcoma cells and in the PBMC of the blood donor, confirming that F3BG10 hybridoma was a DLC/tumor cell hybridoma.

The goal of the second experiment was to investigate whether it was a T-lymphocyte or a B-lymphocyte that fused with a 143B tumor cell to yield the F3BG10 hybridoma. Genomic DNA was tested for the presence of rearranged T-Cell Receptor (TCR) genes or B-cell Receptor (BCR) genes by Southern blot analysis with TCR-specific or BCR-specific probes. Standard procedures were used. Briefly, samples of 10 µg of DNA were submitted to overnight digestion at 37°C with different restricition enzymes. Hind3, Xba1 and Hind3 + Xba1 were used for the TCR rearrangements and EcoR1, Hind3 and Hind3 + BamH1 were used for BCR rearrangements. The restriction fragments were separated by electrophoresis on a 1% agarose gel, transferred to nitrocellulose, baked and hybridized with probes specific for either the β chain gene of the TCR, or for a segment of the J gene of the Ig heavy chain of B lymphocytes. The results clearly showed that there were only germ line TCR genes and germ line BCR genes in the genomic DNA of the F3BG10 hybridoma. These data excluded that the DLC/tumor cell hybridoma F3BG10 was produced by the fusion of the tumor cell with aT-lymphocyte or a B-lymphocyte. The DLC fusion partner could have been a monocyte, a dendritic cell, an intermediate cell between these two cells, a natural killer cell or another unidentified non-B cell. Because the pattern of cytokine secretion could provide indications on the cell lineage of the fusion partner, we investigated cytokine secretion by F3BG10 cells.

### Example 11

### In vitro analysis of cytokine secretion by human DLC/tumor cell hybridoma

The culture supernatants of the F3BG10 hybridoma cells and of the 143B tumor cells were assayed by ELISA for the presence of various cytokines, before and after 36 hours of culture in the presence of various stimuli including interferon γ, TNFα, GM-CSF and combinations of these. The results showed that the 143B osteosarcoma cells and the F3BG10 hybridoma cells secreted similar levels of IL-6 and IL-8, that could be increased for both cytokines by stimulation with the above-mentioned cytokines. In addition, the F3BG10 cells but not the tumor cells secreted significant levels of GM-CSF, that could be increased by stimulation. Neither the tumor cells or the hybridoma cells secreted detectable levels of IL-1β, IL-10, IL-12 and TNFα. These results showed that the F3BG10 hybridoma secreted IL-6 and IL-8 like the parent tumor cell, and GM-CSF like the parent DLC. Since it was excluded that the latter was a T-lymphocyte, this result suggested that the fusion partner was a monocyte.

## Claims

1. Use of a plurality of dendritic cell/tumor cell hybrids for the preparation of a medicament for producing an anti-tumor response in a mammalian subject.

2. Use of a dendritic cell/tumor cell hybridoma for the preparation of a medicament for producing an anti-tumor response in a mammalian subject.

3. Use according to any of claims 1 to 2, wherein the dendritic cell and/or the tumor cell is human in origin.

4. Use according to any of claims 1 to 2, wherein said dendritic cell is derived from bone marrow.

5. Use according to any of claims 1 to 2, wherein said dendritic cell is of myeloid origin.

6. Use according to any of claims 1 to 2, wherein said dendritic cell is of lymphoid origin.

7. Use according to any of claims 1 to 2, wherein said dendritic cell is an isolated dendritic cell.

8. Use according to any of claims 1 to 2, wherein said dendritic cell is a dendritic cell progenitor.

9. Use according to any of claims 1 to 8, wherein said dendritic cell/tumor hybrids are produced using a method comprising:
(a) providing a sample of a tumor against which said response is needed,
(b) preparing a primary cell culture comprising tumor cells derived from said tumor sample,
(c) providing autologous, HLA-compatible or allogeneic dendritic cells, and,
(d) fusing said dendritic cells with said tumor cells to produce a plurality of hybrids.

10. Use according to claim 9, wherein the dendritic cells of step (c) are produced by culturing precursors in the presence of cytokines.

11. Use according to claim 9, wherein the dendritic cells of step (c) are members of an immortal cell line.

12. Use according to any of claims 1 to 8, wherein said dendritic cell/tumor hybridoma is produced using a method comprising:
(a) providing a sample of a tumor against which said response is needed,
(b) preparing a primary culture of said tumor sample to provide tumor cells,
(c) deriving an immortal cell line from said tumor cells to produce immortal tumor cells,
(d) providing autologous or HLA-compatible allogeneic dendritic cells,
(e) fusing said dendritic cells with said immortal tumor cells to produce a plurality of hybridomas,
(f) selecting from said plurality of hybridomas a hybridoma which exhibits at least one characteristic selected from the group consisting of dendritic cell morphology, dendritic-like cell or dendritic cell surface markers, dendritic cell genetic markers and immune cell activation *in vitro*.

13. Use according to claim 12, wherein the dendritic cells of step (d) are produced by culturing precursors in the presence of cytokines.

14. Use according to claim 12 or 13, wherein the immortal tumor cells of step (c) are drug-sensitive, said method further comprising, after step (e), killing unfused drug-sensitive immortal tumor cells by exposure to said drug.

15. Use according to any of claims 1 to 8, wherein said dendritic cell/tumor hybridoma is produced using a method comprising:
(a) providing a sample of a tumor against which said response is needed,
(b) preparing a primary cell culture comprising tumor cells derived from said tumor sample,
(c) providing an immortal cell line comprising immortal autologous or HLA-compatible allogeneic dendritic cells,
(d) fusing said immortal dendritic cells with said tumor cells to produce a plurality of hybridomas, and
(e) selecting from said plurality of hybridomas, a hybridoma which exhibits at least one characteristic selected from the group consisting of tumor cell morphology, tumor cell surface markers, tumor cell chromosomal and genetic markers.

16. Use according to claim 15, wherein said method further comprises a step for selecting from said plurality of hybridomas, a hybridoma which exhibits at least one characteristic selected from the group consisting of dendritic cell morphology, dendritic cell surface markers, dendritic cell genetic markers and immune cell activation *in vitro*.

17. Use according to claim 15 or 16, wherein the dendritic cells of step (c) are drug sensitive, said method further comprising, after step (d), killing unfused drug-sensitive immortal dendritic cells by exposure to said drug.

18. Use according to any of claims 1 to 8, wherein said dendritic cell/tumor hybridoma is produced using a method comprising:
(a) providing a sample of a tumor against which said response is needed,
(b) analyzing tumor-associated antigens of said tumor sample,
(c) providing an established cell line comprising immortal human tumor cells having at least one tumor-associated antigen in common with said tumor sample,
(d) providing autologous, HLA-compatible or allogeneic dendritic cells,
(e) fusing said dendritic cells with said immortal tumor cells to produce a plurality of hybridomas, and
(f) selecting from said plurality of hybridomas, a hybridoma which exhibits at least one characteristic selected from the group consisting of dendritic cell morphology, dendritic cell surface markers, dendritic cell genetic markers and immune cell activation *in vitro.*

19. Use according to claim 18, wherein said method further comprises a step for selecting from said plurality of hybridomas, a hybridoma which expresses at least one tumor-associated antigen in common between the immortal tumor cells and the tumor against which an immune response is needed.

20. Use according to claim 18 or 19, wherein the dendritic cells of step (d) are produced by culturing in the presence of cytokines.

21. Use according to any of claims 18 to 20, wherein the tumor cells of step (c) are drug sensitive, said method comprising, after step (e), killing unfused drug-sensitive tumor cells by exposure to said drug.

22. Use according to any of claims 14, 17 and 21, wherein said drug is hypoxanthine-aminopterin-thymidine (HAT).

23. Use according to any of claims 9 to 11, wherein the obtained hybrid is further induced to express the dendritic cell characteristics.

24. Use according to any of claims 12 to 22, wherein the obtained hybridoma is further induced to express the dendritic cell characteristics.

25. Use according to claim 23 or 24, wherein said dendritic cell characteristics are chosen from the group consisting of dendritic cell morphology, dendritic cell surface markers or dendritic cell activation markers and immune cell activation properties *in vitro*.

26. Use according to any of claims 23 to 25, wherein said induction is performed using GM-CSF.

27. Use according to any of claims 9 to 11, 23, 25 and 26, wherein the obtained hybrid is treated to prevent proliferation before using it for the induction of an anti-tumor response.

28. Use according to any of claims 12 to 22 and 24 to 26, wherein the obtained hybridoma is treated to prevent proliferation before using it for the induction of an anti-tumor response.

29. Use according to claim 27 or 28, wherein said treatment occurs by irradiation.

## Patentansprüche

1. Verwendung einer Vielzahl dendritischer Zell-/Tumorzell-Hybride für die Herstellung eines Medikaments zur Erzeugung einer Anti-Tumor-Reaktion in einem Säugetier.

2. Verwendung eines dendritischen Zell-/Tumorzell-Hybridoms für die Herstellung eines Medikaments zur Erzeugung einer Anti-Tumor-Reaktion in einem Säugetier.

3. Verwendung gemäß einem der Ansprüche 1 bis 2, wobei die dendritische Zelle und/oder die Tumorzelle humanen Ursprungs ist.

4. Verwendung gemäß einem der Ansprüche 1 bis 2, wobei die dendritische Zelle aus Knochenmark stammt.

5. Verwendung gemäß einem der Ansprüche 1 bis 2, wobei die dendritische Zelle myeloiden Ursprungs ist.

6. Verwendung gemäß einem der Ansprüche 1 bis 2, wobei die dendritische Zelle lymphoiden Ursprungs ist.

7. Verwendung gemäß einem der Ansprüche 1 bis 2, wobei die dendritische Zelle eine isolierte dendritische Zelle ist.

8. Verwendung gemäß einem der Ansprüche 1 bis 2, wobei die dendritische Zelle eine dendritische Vorläuferzelle ist.

9. Verwendung gemäß einem der Ansprüche 1 bis 8, wobei die dendritischen Zell-/Tumor-Hybride mit einem Verfahren erzeugt werden, wobei das Verfahren die folgenden Schritte umfasst:
(a) Bereitstellen einer Probe eines Tumors, gegen den die Reaktion benötigt wird,
(b) Herstellen einer primären Zellkultur, die Tumorzellen umfasst, die von der Tumorprobe stammen,
(c) Bereitstellen autologer, HLA-kompatibler oder allogener dendritischer Zellen und,
(d) Fusionieren der dendritischen Zellen mit den Tumorzellen, um eine Vielzahl von Hybriden zu erzeugen.

10. Verwendung gemäß Anspruch 9, wobei die dendritischen Zellen aus Schritt (c) erzeugt werden, indem Vorläufer in Gegenwart von Zytokinen kultiviert werden.

11. Verwendung gemäß Anspruch 9, wobei die dendritischen Zellen aus Schritt (c) Mitglieder einer immortalisierten Zelllinie sind.

12. Verwendung gemäß einem der Ansprüche 1 bis 8, wobei das dendritische Zell-/Tumor-Hybridom mit einem Verfahren erzeugt wird, wobei das Verfahren die folgenden Schritte umfasst:
(a) Bereitstellen einer Probe eines Tumors, gegen den die Reaktion benötigt wird,
(b) Herstellen einer primären Kultur der Tumorprobe, um Tumorzellen bereitzustellen,
(c) Ableiten einer immortalisierten Zelllinie von den Tumorzellen, um immortalisierte Tumorzellen zu erzeugen,
(d) Bereitstellen autologer oder HLA-kompatibler allogener dendritischer Zellen,
(e) Fusionieren der dendritischen Zellen mit den immortalisierten Tumorzellen, um eine Vielzahl von Hybridomen zu erzeugen,
(f) Auswählen eines Hybridoms aus der Vielzahl von Hybridomen, wobei das Hybridom wenigstens eine Eigenschaft aufweist, die aus dendritischer Zellmorphologie, aus Oberflächenmarkern von Zellen, die dendritischen Zellen ähneln, oder aus Oberflächenmarkern von dendritischen Zellen, aus genetischen Markern dendritischer Zellen und aus *In vitro*-Immunzellaktivierung ausgewählt wird.

13. Verwendung gemäß Anspruch 12, wobei die dendritischen Zellen aus Schritt (d) erzeugt werden, indem Vorläufer in Gegenwart von Zytokinen kultiviert werden.

14. Verwendung gemäß Anspruch 12 oder 13, wobei die immortalisierten Tumorzellen aus Schritt (c) Wirkstoff-sensitiv sind, und wobei das Verfahren weiter, nach Schritt (e), den Schritt umfasst, nicht-fusionierte Wirkstoff-sensitive immortalisierte Tumorzellen abzutöten, indem sie dem Wirkstoff ausgesetzt werden.

15. Verwendung gemäß einem der Ansprüche 1 bis 8, wobei das dendritische Zell-/Tumor-Hybridom mit einem Verfahren erzeugt wird, wobei das Verfahren die folgenden Schritte umfasst:
(a) Bereitstellen einer Probe eines Tumors, gegen den die Reaktion benötigt wird,
(b) Herstellen einer primären Zellkultur, die Tumorzellen umfasst, die von der Tumorprobe stammen,
(c) Bereitstellen einer immortalisierten Zelllinie, die immortalisierte autologe oder HLA-kompatible allogene dendritische Zellen umfasst,
(d) Fusionieren dieser immortalisierten dendritischen Zellen mit den Tumorzellen, um eine Vielzahl von Hybridomen zu erzeugen und
(e) Auswählen eines Hybridoms aus der Vielzahl von Hybridomen, wobei das Hybridom wenigstens eine Eigenschaft aufweist, die aus Tumorzellmorphologie, aus Oberflächenmarkern von Tumorzellen, aus chromosomalen und genetischen Markern von Tumorzellen ausgewählt wird.

16. Verwendung gemäß Anspruch 15, wobei das Verfahren weiter einen Schritt für die Auswahl eines Hybridoms aus der Vielzahl von Hybridomen umfasst, wobei das Hybridom wenigstens eine Eigenschaft aufweist, die aus dendritischer Zellmorphologie, aus Oberflächenmarkern von dendritischen Zellen, aus genetischen Markern dendritischer Zellen und aus *In vitro*-Immunzellaktivierung ausgewählt wird.

17. Verwendung gemäß Anspruch 15 oder 16, wobei die dendritischen Zellen aus Schritt (c) Wirkstoff-sensitiv sind, und wobei das Verfahren weiter, nach Schritt (d), den Schritt umfasst, nicht-fusionierte Wirkstoff-sensitive immortalisierte dendritische Zellen abzutöten, indem sie dem Wirkstoff ausgesetzt werden.

18. Verwendung gemäß einem der Ansprüche 1 bis 8, wobei das dendritische Zell-/Tumor-Hybridom mit einem Verfahren erzeugt wird, wobei das Verfahren die folgenden Schritte umfasst:
(a) Bereitstellen einer Probe eines Tumors, gegen den die Reaktion benötigt wird,
(b) Untersuchen von Tumor-assoziierten Antigenen der Tumorprobe,
(c) Bereitstellen einer etablierten Zelllinie, die immortalisierte humane Tumorzellen mit wenigstens einem Tumor-assoziierten Antigen umfasst, das sie mit der Tumorprobe gemeinsam haben,
(d) Bereitstellen autologer, HLA-kompatibler oder allogener dendritischer Zellen,
(e) Fusionieren der dendritischen Zellen mit den immortalisierten Tumorzellen, um eine Vielzahl von Hybridomen zu erzeugen und
(f) Auswählen eines Hybridoms aus der Vielzahl von Hybridomen, wobei das Hybridom wenigstens eine Eigenschaft aufweist, die aus dendritischer Zellmorphologie, aus Oberflächenmarkern von dendritischen Zellen, aus genetischen Markem dendritischer Zellen und aus *In vitro*-Immunzellaktivierung ausgewählt wird.

19. Verwendung gemäß Anspruch 18, wobei das Verfahren weiter einen Schritt für die Auswahl eines Hybridoms aus der Vielzahl von Hybridomen umfasst, wobei das Hybridom wenigstens ein Tumor-assoziiertes Antigen exprimiert, welches die immortalisierten Tumorzellen und der Tumor, gegen den eine Immunreaktion benötigt wird, gemeinsam haben.

20. Verwendung gemäß Anspruch 18 oder 19, wobei die dendritischen Zellen aus Schritt (d) durch Kultivierung in Gegenwart von Zytokinen erzeugt werden.

21. Verwendung gemäß Anspruch 18 bis 20, wobei die Tumorzellen aus Schritt (c) Wirkstoff-sensitiv sind, und wobei das Verfahren, nach Schritt (e), den Schritt umfasst, nicht-fusionierte Wirkstoff-sensitive immortalisierte Tumorzellen abzutöten, indem sie dem Wirkstoff ausgesetzt werden.

22. Verwendung gemäß einem der Ansprüche 14, 17 und 21, wobei der Wirkstoff Hypoxanthin-Aminopterin-Thymidin (HAT) ist.

23. Verwendung gemäß einem der Ansprüche 9 bis 11, wobei das erhaltene Hybrid weiter induziert wird, so dass es die Eigenschaften dendritischer Zellen exprimiert.

24. Verwendung gemäß einem der Ansprüche 12 bis 22, wobei das erhaltene Hybridom weiter induziert wird, so dass es die Eigenschaften dendritischer Zellen exprimiert.

25. Verwendung gemäß Anspruch 23 oder 24, wobei die Eigenschaften dendritischer Zellen aus dendritischer Zellmorphologie, aus Oberflächenmarkern von dendritischen Zellen oder Aktivierungsmarkern von dendritischen Zellen und Eigenschaften der *In vitro*-Immunzellaktivierung ausgewählt werden.

26. Verwendung gemäß einem der Ansprüche 23 bis 25, wobei die Induktion mit GM-CSF ausgeführt wird.

27. Verwendung gemäß einem der Ansprüche 9 bis 11, 23, 25 und 26, wobei das erhaltene Hybrid behandelt wird, so dass eine Poliferation verhindert wird bevor es für die Induktion einer Anti-Tumor-Reaktion verwendet wird.

28. Verwendung gemäß einem der Ansprüche 12 bis 22 und 24 bis 26, wobei das erhaltene Hybridom behandelt wird, so dass eine Poliferation verhindert wird bevor es für die Induktion einer Anti-Tumor-Reaktion verwendet wird.

29. Verwendung gemäß Anspruch 27 oder 28, wobei die Behandlung durch Bestrahlung erfolgt.

## Revendications

1. Utilisation d'une pluralité d'hybrides de cellules dendritiques/cellules tumorales pour la préparation d'un médicament destiné à la production d'une réponse anti-tumorale chez un sujet mammifère.

2. Utilisation d'un hybridome de cellules dendritiques/cellules tumorales pour la préparation d'un médicament destiné à produire une réponse anti-tumorale chez un sujet mammifère.

3. Utilisation selon l'une quelconque des revendications 1 à 2, dans laquelle la cellule dendritique et/ou la cellule tumorale sont d'origine humaine.

4. Utilisation selon l'une quelconque des revendications 1 à 2, dans laquelle ladite cellule dendritique est dérivée de moelle osseuse.

5. Utilisation selon l'une quelconque des revendications 1 à 2, dans laquelle ladite cellule dendritique est d'origine myéloïde.

6. Utilisation selon l'une quelconque des revendications 1 à 2, dans laquelle ladite cellule dendritique est d'origine lymphoïde.

7. Utilisation selon l'une quelconque des revendications 1 à 2, dans laquelle ladite cellule dendritique est une cellule dendritique isolée.

8. Utilisation selon l'une quelconque des revendications 1 à 2, dans laquelle ladite cellule dendritique est un progéniteur de cellule dendritique.

9. Utilisation selon l'une quelconque des revendications 1 à 8, dans laquelle lesdits hybrides de cellules dendritiques/cellules tumorales sont produits en utilisant un procédé comprenant :
(a) la fourniture d'un échantillon d'une tumeur contre laquelle ladite réponse est nécessaire,
(b) la préparation d'une culture primaire de cellules comprenant des cellules tumorales dérivées dudit échantillon de tumeur,
(c) la fourniture de cellules dendritiques allogéniques, autologues ou HLA compatibles, et
(d) la fusion desdites cellules dendritiques avec lesdites cellules tumorales pour produire une pluralité d'hybrides.

10. Utilisation selon la revendication 9, dans laquelle les cellules dendritiques de l'étape (c) sont produites par la culture de précurseurs en présence de cytokines.

11. Utilisation selon la revendication 9, dans laquelle les cellules dendritiques de l'étape (c) sont des membres d'une lignée cellulaire immortelle.

12. Utilisation selon l'une quelconque des revendications 1 à 8, dans laquelle ledit hybridome de cellules dendritiques/cellules tumorales est produit en utilisant un procédé comprenant :
(a) la fourniture d'un échantillon d'une tumeur contre laquelle ladite réponse est nécessaire,
(b) la préparation d'une culture primaire dudit échantillon de tumeur pour fournir des cellules tumorales,
(c) l'obtention d'une lignée cellulaire immortelle desdites cellules tumorales pour produire des cellules tumorales immortelles,
(d) la fourniture de cellules dendritiques allogéniques autologues ou HLA compatibles,
(e) la fusion desdites cellules dendritiques avec lesdites cellules tumorales immortelles pour produire une pluralité d'hybridomes,
(f) la sélection, dans ladite pluralité d'hybridomes, d'un hybridome qui présente au moins une caractéristique choisie dans le groupe constitué par la morphologie des cellules dendritiques, les marqueurs de surface des cellules de type dendritique ou des cellules dendritiques, les marqueurs génétiques des cellules dendritiques et l'activation des cellules immunitaires *in vitro*.

13. Utilisation selon la revendication 12, dans laquelle les cellules dendritiques de l'étape (d) sont produites par la culture de précurseurs en présence de cytokines.

14. Utilisation selon la revendication 12 ou 13, dans laquelle les cellules tumorales immortelles de l'étape (c) sont sensibles aux médicaments, ledit procédé comprenant en outre, après l'étape (e), l'élimination des cellules tumorales immortelles, sensibles aux médicaments et non fusionnées, par exposition au dit médicament.

15. Utilisation selon l'une quelconque des revendications 1 à 8, dans laquelle ledit hybridome de cellules dendritiques/cellules tumorales est produit en utilisant un procédé comprenant :
(a) la fourniture d'un échantillon d'une tumeur contre laquelle ladite réponse est nécessaire,
(b) la préparation d'une culture primaire de cellules comprenant des cellules tumorales dérivées dudit échantillon de tumeur,
(c) la fourniture d'une lignée cellulaire immortelle comprenant des cellules dendritiques allogéniques autologues ou HLA compatibles,
(d) la fusion desdites cellules dendritiques immortelles avec lesdites cellules tumorales pour produire une pluralité d'hybridomes, et
(e) la sélection, dans ladite pluralité d'hybridomes, d'un hybridome qui présente au moins une caractéristique choisie dans le groupe const.itué par la morphologie des cellules tumorales, les marqueurs de surface des cellules tumorales, les marqueurs chromosomiques et génétiques des cellules tumorales.

16. Utilisation selon la revendication 15, dans laquelle ledit procédé comprend en outre une étape de sélection dans ladite pluralité d'hybridomes d'un hybridome qui présente au moins une caractéristique choisie dans le groupe constitué par la morphologie des cellules dendritiques, les marqueurs de surface des cellules dendritiques, les marqueurs génétiques des cellules dendritiques et l'activation des cellules immunitaires in vitro.

17. Utilisation selon la revendication 15 ou 16, dans laquelle les cellules dendritiques de l'étape (c) sont sensibles aux médicaments, ledit procédé comprenant en outre, après l'étape (d), l'élimination des cellules dendritiques immortelles, sensibles aux médicaments et non fusionnées par exposition au dit médicament.

18. Utilisation selon l'une quelconque des revendications 1 à 8, dans laquelle ledit hybridome de cellules dendritiques/cellules tumorales est produit en utilisant un procédé comprenant :
(a) la fourniture d'un échantillon d'une tumeur contre laquelle ladite réponse est nécessaire,
(b) l'analyse des antigènes associés à la tumeur dudit échantillon de tumeur,
(c) la fourniture d'une lignée cellulaire établie comprenant des cellules tumorales et immortelles humaines ayant au moins un antigène associé à la tumeur en commun avec ledit échantillon de tumeur,
(d) la fourniture de cellules dendritiques allogéniques, autologues ou HLA compatibles,
(e) la fusion desdites cellules dendritiques avec lesdites cellules tumorales immortelles pour produire une pluralité d'hybridomes, et
(f) la sélection dans ladite pluralité d'hybridomes, d'un hybridome qui présente au moins une caractéristique choisie dans le groupe constitué par la morphologie des cellules dendritiques, les marqueurs de surface des cellules dendritiques, les marqueurs génétiques des cellules dendritiques et l'activation des cellules immunitaires *in vitro*.

19. Utilisation selon la revendication 18, dans laquelle ledit procédé comprend en outre une étape de sélection dans ladite pluralité d'hybridomes, d'un hybridome qui exprime au moins un antigène associé à la tumeur en commun avec les cellules tumorales immortelles et la tumeur contre laquelle une réponse immunitaire est nécessaire.

20. Utilisation selon la revendication 18 ou 19, dans laquelle les cellules dendritiques de l'étape (d) sont produites par culture en présence de cytokines.

21. Utilisation selon l'une quelconque des revendications 18 à 20, dans laquelle les cellules tumorales de l'étape (c) sont sensibles aux médicaments, ledit procédé comprenant, après l'étape (e), l'élimination des cellules tumorales, sensibles aux médicaments et non fusionnées par exposition au dit médicament.

22. Utilisation selon l'une quelconque des revendications 14, 17 et 21, dans laquelle ledit médicament est l'hypoxanthine-aminoptérine-thymidine (HAT).

23. Utilisation selon l'une quelconque des revendications 9 à 11, dans laquelle l'hydride obtenu est ensuite induit pour exprimer les caractéristiques des cellules dendritiques.

24. Utilisation selon l'une quelconque des revendications 12 à 22, dans laquelle l'hybridome obtenu est ensuite induit pour exprimer les caractéristiques des cellules dendritiques.

25. Utilisation selon la revendication 23 ou 24, dans laquelle lesdites caractéristiques des cellules dendritiques sont choisies dans le groupe constitué par la morphologie des cellules dendritiques, les marqueurs de surface des cellules dendritiques ou les marqueurs d'activation des cellules dendritiques et les propriétés d'activation des cellules immunitaires *in vitro*.

26. Utilisation selon l'une quelconque des revendications 23 à 25, dans laquelle ladite induction est réalisée en utilisant le GM-CSF.

27. Utilisation selon l'une quelconque des revendications 9 à 11, 23, 25 et 26, dans laquelle l'hydride obtenu est traité pour empêcher la prolifération avant son utilisation pour l'induction d'une réponse anti-tumorale.

28. Utilisation selon l'une quelconque des revendications 12 à 22 et 24 à 26, dans laquelle l'hybridome obtenu est traité pour empêcher la prolifération avant son utilisation pour l'induction d'une réponse anti-tumorale.

29. Utilisation selon la revendication 27 ou 28, dans laquelle ledit traitement est réalisé par irradiation.
